# EUROPÄISCHE PATENTSCHRIFT

(11) **EP 3 426 773 B1**
(45) Veröffentlichungstag und Bekanntmachung des Hinweises auf die Patenterteilung: **11.11.2020**
(21) Anmeldenummer: 17709432.3
(22) Anmeldetag: 07.03.2017
(51) Int. Cl.: C12N 9/10

(54) **SACCHAROSE PHOSPHORYLASE**
SACCHAROSE PHOSPHORYLASIS
SACCHAROSE PHOSPHORYLASE

(30) Priorität: 08.03.2016 EP 16159122
(43) Veröffentlichungstag der Anmeldung: 16.01.2019
(73) Patentinhaber: Pfeifer & Langen GmbH & Co. KG, 50858 Köln (DE)
(72) Erfinder: KOCH, Timo Johannes, 50189 Elsdorf (DE); HÄSSLER, Thomas, 50968 Köln (DE); BRUCHER, Birgit, 04275 Leipzig (DE); VOGEL, Andreas, 04105 Leipzig (DE)
(74) Vertreter: Kutzenberger Wolff & Partner
(86) Internationale Anmeldenummer: PCT/EP2017/055347
(87) Internationale Veröffentlichungsnummer: WO 2017/153420

(56) Entgegenhaltungen:
- WO-A1-2011/124538
- DATABASE UniProt [Online] 29. Oktober 2014 (2014-10-29), "SubName: Full=Trey {ECO:0000313|EMBL:KFJ05600.1}; EC=2.4.1.7 {ECO:0000313|EMBL:KFJ05600.1};", XP002769991, gefunden im EBI accession no. UNIPROT:A0A087ECU9 Database accession no. A0A087ECU9
- TOM VERHAEGHE ET AL: "Mapping the acceptor site of sucrose phosphorylase from Bifidobacterium adolescentis by alanine scanning", JOURNAL OF MOLECULAR CATALYSIS B: ENZYMATIC, Bd. 96, 1. Dezember 2013 (2013-12-01), Seiten 81-88, XP055166136, ISSN: 1381-1177, DOI: 10.1016/j.molcatb.2013.06.014
- A. CERDOBBEL ET AL: "Increasing the thermostability of sucrose phosphorylase by a combination of sequence- and structure-based mutagenesis", PROTEIN ENGINEERING DESIGN AND SELECTION, Bd. 24, Nr. 11, 1. November 2011 (2011-11-01), Seiten 829-834, XP055166150, ISSN: 1741-0126, DOI: 10.1093/protein/gzr042

## Beschreibung

Die Erfindung betrifft eine Saccharose-Phosphorylase, welche u.a. die Synthese von Glucose-1-Phosphat und Fructose aus Saccharose und Phosphat katalysiert. Die erfindungsgemäße Saccharose-Phosphorylase kann als Mutante der Saccharose-Phosphorylase aus *Bifidobacterium adolescentis* aufgefasst werden. Im Vergleich zur Saccharose-Phosphorylase des Wildtyps zeichnet sich die erfindungsgemäße Saccharose-Phosphorylase durch eine verbesserte Aktivität, Prozessstabilität, Temperaturstabilität, sowie eine geringere Produktinhibition aus und ist daher in besonderem Maße zum Einsatz in industriellen Prozessen geeignet.

Saccharose-Phosphorylasen katalysieren die Umsetzung von Saccharose und Phosphat zu Fructose und Glucose-1-Phosphat. Neben dieser phosphorolytischen Aktivität sind die Enzyme unter geeigneten Bedingungen auch in der Lage, umgekehrt die Synthese von Saccharose aus Glucose-1-Phosphat und Fructose unter Freisetzung von Phosphat zu katalysieren.

Cellobiose ist ein natürliches Disaccharid, welches den Grundbaustein für Cellulose bildet. Cellobiose gewinnt zunehmend an Attraktivität für den Lebens- und Futtermittelbereich. Die Herstellung von Cellobiose kann über chemische oder enzymatische Hydrolyse von Cellulose erfolgen. GB 2 438 573 beschreibt ein Verfahren zur Gewinnung von Cellobiose, welches die Hydrolyse von Cellulose, die Erhöhung des Cellobiose-Anteils in der Zuckerlösung relativ zu weiteren Sacchariden durch Ultrafiltration, sowie anschließende Reinigung der Cellobiose durch Kristallisation umfasst.

Eine Alternative zur Herstellung von Cellobiose aus Cellulose ist die Synthese von Cellobiose unter Verwendung von Cellobiose-Phosphorylasen. Dabei kann Glucose-1-Phosphat als Intermediat entweder durch Phosphorolyse von Stärke mittels einer alpha-Glukan-Phosphorylase oder durch Phosphorolyse von Saccharose mittels einer Saccharose-Phosphorylase erhalten werden.

EP 0 423 768 offenbart ein Verfahren zur Herstellung von Cellobiose ausgehend von Saccharose unter Verwendung einer Saccharose-Phosphorylase, einer Glucose-Isomerase und einer Cellobiose-Phosphorylase. Das Verfahren umfasst folgende Schritte: (1) Spaltung der Saccharose in Anwesenheit von Orthophosphat unter Katalyse von Saccharose-Phosphorylase in Glucose-1-Phosphat und Fructose; (2) Isomerisierung von Fructose in Glucose unter Katalyse von Glucose-Isomerase; (3) Synthese von Cellobiose aus Glucose und Glucose-1-Phosphat unter Katalyse von Cellobiose-Phosphorylase unter Abspaltung von Orthophosphat; (4) teilweise Aufarbeitung der Cellobiose aus dem Reaktionsansatz sowie Rückführung eines Teils des verbleibenden Orthophosphat-haltigen Reaktionsansatzes in Schritt (1).

WO 2011/124538 A1 offenbart eine Variante der Saccharose-Phosphorylase aus *Bifidobacterium adolescentis,* welche erhöhte Stabilität aufweist und für 16 Stunden bei 60 °C enzymatisch aktiv ist. Des Weiteren werden Verfahren beschrieben, mit denen das Enzym exprimiert und zur Synthese von Glucose-1-Phosphat und Fructose verwendet wird.

Verhaeghe et al., "Mapping the acceptor site of sucrose phosphorylase from Bifidobacterium adolescensis by alanin scanning", Journal of Molecular Catalysis B: Enzymatic, Bd. 96, 1.12.2013, S. 81-88, beschreibt die Untersuchung des Wildtyps der Saccharose-Phosphorylase aus *Bifidobacterium adolescensis* im Hinblick auf ihre Affinität für verschiedene Substrate. Es werden verschiedene Stellen in der Aminosäuresequenz der Saccharose-Phosphorylase offenbart, die Einfluss auf die Substrataffinität haben.

Cerdobbel et al., "Increasing the thermostability of sucrose phosphorylase by a combination of sequence- and structure-based mutagenesis", Protein Engineering Design and Selection, Bd. 24, Nr. 11, 1.11.2011, S. 829-834, offenbart eine Variante der Saccharose-Phosphorylase aus *Bifidobacterium adolescentis* mit sechs Mutationen in der Aminosäuresequenz, die eine verbesserte Temperaturstabilität bei 60 ° C hat.

Die Datenbanken DATABASE UniProt, ID A0A087AXC6; DATABASE Geneseq, SEQ ID 323, DATABASE UniProt, ID A0A087CXQ8 und DATABASE UniProt, ID A0A087CMV6 offenbaren Mutationen in der Aminosäuresequenzen der Saccharose-Phosphorylase aus *Bifidobacterium adolescensis.*

Das Schlüsselintermediat dieser Synthesen von Cellobiose ist Glucose-1-Phosphat. Glucose-1-Phosphat wird bei vielen biochemischen Prozessen als Intermediat durchlaufen und spielt daher im Stoffwechsel eine zentrale Rolle.

Eine Möglichkeit zur Optimierung von Enzymen besteht in der Anwendung von Enzym-Engineering, welches auf die Entwicklung von Varianten eines Ausgangsenzyms mit verbesserten Eigenschaften abzielt. Enzym-Engineering wurde bereits auf eine Saccharose-Phosphorylase aus *Bifidobacterim adolescentis* angewandt. WO 2011/124538 betrifft eine Saccharose-Phosphorylase aus *Bifidobacterium adolescentis,* welche als Biokatalysator zur Umwandlung von Kohlenhydraten bei erhöhter Temperatur geeignet ist.

Es besteht ein Bedarf an verbesserten Verfahren zur Herstellung von Glucose-1-Phosphat im industriellen Maßstab, vorzugsweise aus Saccharose und Phosphat als Ausgangsmaterial, damit dieses Glucose-1-Phosphat dann anschließend in weiteren Umsetzungen eingesetzt werden kann, insbesondere zur Synthese von Cellobiose unter Katalyse einer Cellobiose-Phosphorylase.

Der Erfindung liegt die Aufgabe zugrunde, eine Saccharose-Phosphorylase mit verbesserten Eigenschaften bereitzustellen. Im Hinblick auf die Synthese von Glucose-1-Phosphat aus Saccharose und Phosphat sollte sich die Saccharose-Phosphorylase im Vergleich zu bekannten Saccharose-Phosphorylasen möglichst durch eine verbesserte Aktivität und Prozessstabilität, insbesondere Temperaturstabilität, sowie eine geringere Edukt- und Produktinhibition auszeichnen.

Diese Aufgabe wird durch den Gegenstand der Patentansprüche gelöst.

Offenbart wird eine Saccharose-Phosphorylase umfassend eine Aminosäuresequenz, welche eine Identität zur Aminosäuresequenz gemäß SEQ ID NO:1 von mindestens 80%, mindestens 81%, mindestens 82%, mindestens 83%, oder mindestens 84%, bevorzugter mindestens 85%, mindestens 86%, mindestens 87%, mindestens 88%, oder mindestens 89%, noch bevorzugter mindestens 90%, mindestens 91%, mindestens 92%, mindestens 93%, oder mindestens 94%, am bevorzugtesten mindestens 95%, mindestens 96%, mindestens 97%, oder mindestens 98%, und insbesondere mindestens 98,5%, mindestens 99,0%, mindestens 99,4%, mindestens 99,6% oder mindestens 99,8% aufweist, und welche im Vergleich zu SEQ ID NO:1 wenigstens eine Aminosäure-Mutation, bevorzugt wenigstens zwei, wenigstens drei oder wenigstens vier Aminosäure-Mutationen umfasst jeweils unabhängig voneinander
- in einem Sequenzabschnitt A), welcher Positionen 11 bis 31 gemäß SEQ ID NO:1 entspricht; und/oder
- in einem Sequenzabschnitt B), welcher Positionen 132 bis 161 gemäß SEQ ID NO:1 entspricht; und/oder
- in einem Sequenzabschnitt C), welcher Positionen 175 bis 195 gemäß SEQ ID NO:1 entspricht; und/oder
- in einem Sequenzabschnitt D), welcher Positionen 285 bis 305 gemäß SEQ ID NO:1 entspricht; und/oder
- in einem Sequenzabschnitt E), welcher Positionen 394 bis 406 gemäß SEQ ID NO:1 entspricht; und/oder
- in einem Sequenzabschnitt F), welcher Positionen 447 bis 459 gemäß SEQ ID NO:1 entspricht; und/oder
- in einem Sequenzabschnitt G), welcher Positionen 464 bis 484 gemäß SEQ ID NO:1 entspricht.

In einer offenbarten Ausführungsform umfasst die Saccharose-Phosphorylase eine Aminosäuresequenz, welche eine Identität zur Aminosäuresequenz gemäß SEQ ID NO:1 von mindestens 80% aufweist und welche im Vergleich zu SEQ ID NO:1
- eine Aminosäure-Mutation in einem Sequenzabschnitt C) umfasst, welcher Positionen 175 bis 195 gemäß SEQ ID NO:1 entspricht; und
- eine Aminosäure-Mutation in einem Sequenzabschnitt D) umfasst, welcher Positionen 285 bis 305 gemäß SEQ ID NO:1 entspricht.

In einer offenbarten Ausführungsform umfasst die Saccharose-Phosphorylase eine Aminosäuresequenz, welche eine Identität zur Aminosäuresequenz gemäß SEQ ID NO:1 von mindestens 80%, mindestens 81%, mindestens 82%, mindestens 83%, oder mindestens 84%, bevorzugter mindestens 85%, mindestens 86%, mindestens 87%, mindestens 88%, oder mindestens 89%, noch bevorzugter mindestens 90%, mindestens 91%, mindestens 92%, mindestens 93%, oder mindestens 94%, am bevorzugtesten mindestens 95%, mindestens 96%, mindestens 97%, oder mindestens 98%, und insbesondere mindestens 98,5%, mindestens 99,0%, mindestens 99,4%, mindestens 99,6% oder mindestens 99,8% aufweist, und welche im Vergleich zu SEQ ID NO:1 wenigstens eine Aminosäure-Mutation, bevorzugt wenigstens zwei, wenigstens drei oder wenigstens vier Aminosäure-Mutationen umfasst jeweils unabhängig voneinander
- in einem Sequenzabschnitt A), welcher Positionen 11 bis 31 gemäß SEQ ID NO:1 entspricht; und/oder
- in einem Sequenzabschnitt C), welcher Positionen 175 bis 195 gemäß SEQ ID NO:1 entspricht; und/oder
- in einem Sequenzabschnitt D), welcher Positionen 285 bis 305 gemäß SEQ ID NO:1 entspricht; und/oder
- in einem Sequenzabschnitt G), welcher Positionen 464 bis 484 gemäß SEQ ID NO:1 entspricht.

Es wurde überraschend gefunden, dass durch Aminosäure-Mutationen in bestimmten Sequenzabschnitten der Aminosäuresequenz der Saccharose-Phosphorylase aus *Bifidobacterium adolescentis* (Wildtyp, SEQ ID NO:1) verbesserte Saccharose-Phosphorylasen erhältlich sind.

Die erfindungsgemäße Saccharose-Phosphorylase zeichnet sich im Vergleich zur Saccharose-Phosphorylase des Wildtyps durch eine verbesserte Aktivität, Prozessstabilität, Temperaturstabilität, sowie eine geringere Edukt- und Produktinhibition aus und ist daher in besonderem Maße zum Einsatz in industriellen Prozessen geeignet. Die verbesserten Eigenschaften können zu einer verbesserten Raum-Zeit-Ausbeute und einem erhöhten Verhältnis von Phosphorolyse zu Synthese führen.

Die erfindungsgemäße Saccharose-Phosphorylase umfasst eine Aminosäuresequenz, welche eine Identität zur Aminosäuresequenz gemäß SEQ ID NO:1 von mindestens 80% aufweist, bevorzugt mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99%.

Erfindungsgemäß ist "Identität" definiert als der Prozentsatz identischer Übereinstimmungen zwischen zwei Vergleichssequenzen bei optimaler Ausrichtung (*alignment*)*.* Zur optimalen Ausrichtung können Lücken in jeder der beiden Sequenzen eingeführt werden. Bevorzugt wird die Identität zwischen zwei Vergleichssequenzen unter Verwendung des Smith and Waterman-Algorithmus bestimmt (Smith TF, Waterman MS J (1981) Mol. Biol. 147, 195-197), bevorzugt unter Verwendung des Computerprogramms WATER aus dem EMBOSS-Paket, welches frei verfügbar ist und den Smith und Waterman-Algorithmus implementiert hat (Reis P, Longden I, Bleasby A (2000) Trends in Genetics 16, 276-277). Dabei wird bevorzugt BLOSUM62 für die Substitutionsmatrix mit einem GOP (*gap opening penalty*) von 10 und einem GEP (*gap extension penalty*) von 0,5 verwendet.

Eine Aminosäure-Mutation im Sinne dieser Erfindung ist definiert als Austausch der Aminosäure einer Saccharose-Phosphorylase Wildtypsequenz, bevorzugt der Saccharose-Phosphorylase Wildtypsequenz gemäß SEQ ID NO:1, in eine andere proteinogene Aminosäure.

In offenbarten Ausführungsformen der Saccharose-Phosphorylase entspricht
- Sequenzabschnitt A) Positionen 16 bis 26 gemäß SEQ ID NO:1; und/oder
- Sequenzabschnitt B) Positionen 137 bis 156 gemäß SEQ ID NO:1; und/oder
- Sequenzabschnitt C) Positionen 180 bis 190 gemäß SEQ ID NO:1; und/oder
- Sequenzabschnitt D) Positionen 290 bis 300 gemäß SEQ ID NO:1; und/oder
- Sequenzabschnitt E) Positionen 394 bis 401 gemäß SEQ ID NO:1; und/oder
- Sequenzabschnitt F) Positionen 447 bis 456 gemäß SEQ ID NO:1; und/oder
- Sequenzabschnitt G) Positionen 469 bis 481 gemäß SEQ ID NO:1.

In offenbarten Ausführungsformen der Saccharose-Phosphorylase entspricht
- Sequenzabschnitt A) Positionen 20 bis 22 gemäß SEQ ID NO:1; und/oder
- Sequenzabschnitt B) Positionen 141 bis 152 gemäß SEQ ID NO:1; und/oder
- Sequenzabschnitt C) Positionen 184 bis 186 gemäß SEQ ID NO:1; und/oder
- Sequenzabschnitt D) Positionen 294 bis 296 gemäß SEQ ID NO:1; und/oder
- Sequenzabschnitt E) Positionen 395 bis 397 gemäß SEQ ID NO:1, und/oder
- Sequenzabschnitt F) Positionen 450 bis 452 gemäß SEQ ID NO:1; und/oder
- Sequenzabschnitt G) Positionen 473 bis 477 gemäß SEQ ID NO:1.

In einer bevorzugten Ausführungsform umfasst die erfindungsgemäße Saccharose-Phosphorylase im Vergleich zu SEQ ID NO:1 wenigstens eine, bevorzugter aber auch nicht mehr als eine Aminosäure-Mutation in dem wie vorstehend definierten Sequenzabschnitt A).

In einer bevorzugten Ausführungsform umfasst die erfindungsgemäße Saccharose-Phosphorylase im Vergleich zu SEQ ID NO:1 wenigstens eine, bevorzugter wenigstens 2, noch bevorzugter aber auch nicht mehr als zwei Aminosäure-Mutationen in dem wie vorstehend definierten Sequenzabschnitt B).

In einer offenbarten Ausführungsform umfasst die Saccharose-Phosphorylase im Vergleich zu SEQ ID NO:1 wenigstens eine, bevorzugter aber auch nicht mehr als eine Aminosäure-Mutation in dem wie vorstehend definierten Sequenzabschnitt C).

In einer offenbarten Ausführungsform umfasst die Saccharose-Phosphorylase im Vergleich zu SEQ ID NO:1 wenigstens eine, bevorzugter aber auch nicht mehr als eine Aminosäure-Mutation in dem wie vorstehend definierten Sequenzabschnitt D).

In einer bevorzugten Ausführungsform umfasst die erfindungsgemäße Saccharose-Phosphorylase im Vergleich zu SEQ ID NO:1 wenigstens eine, bevorzugter aber auch nicht mehr als eine Aminosäure-Mutation in dem wie vorstehend definierten Sequenzabschnitt E).

In einer bevorzugten Ausführungsform umfasst die erfindungsgemäße Saccharose-Phosphorylase im Vergleich zu SEQ ID NO:1 wenigstens eine, bevorzugter aber auch nicht mehr als eine Aminosäure-Mutation in dem wie vorstehend definierten Sequenzabschnitt F).

In einer bevorzugten Ausführungsform umfasst die erfindungsgemäße Saccharose-Phosphorylase im Vergleich zu SEQ ID NO:1 wenigstens eine, bevorzugter wenigstens 2, noch bevorzugter aber auch nicht mehr als zwei Aminosäure-Mutationen in dem wie vorstehend definierten Sequenzabschnitt G).

In bevorzugten Ausführungsformen umfasst die erfindungsgemäße Saccharose-Phosphorylase im Vergleich zu SEQ ID NO:1 wenigstens zwei, drei oder vier Aminosäure-Mutationen.

In einer offenbarten Ausführungsform liegen die wenigstens zwei, drei oder vier Aminosäure-Mutationen unabhängig voneinander in Sequenzabschnitt A), B), C), D), E), F) und/oder G).

In einer offenbarten Ausführungsform liegen die wenigstens zwei, drei oder vier Aminosäure-Mutationen unabhängig voneinander in Sequenzabschnitten A), C), D) und/oder G), wobei bevorzugt eine Aminosäure-Mutation in Sequenzabschnitt A), eine Aminosäure-Mutation in Sequenzabschnitt C), eine Aminosäure-Mutation in Sequenzabschnitt D), und/oder eine Aminosäure-Mutation in Sequenzabschnitt G) liegt.

In einer anderen offenbarten Ausführungsform liegen die wenigstens zwei, drei oder vier Aminosäure-Mutationen unabhängig voneinander in Sequenzabschnitten C), D), F) und/oder G), wobei bevorzugt eine Aminosäure-Mutation in Sequenzabschnitt C), eine Aminosäure-Mutation in Sequenzabschnitt D), eine Aminosäure-Mutation in Sequenzabschnitt F), und/oder eine Aminosäure-Mutation in Sequenzabschnitt G) liegt.

In offenbarten Formen umfasst die Saccharose-Phosphorylase im Vergleich zu SEQ ID NO:1 wenigstens zwei Aminosäure-Mutationen, nämlich *"erste Aminosäure-Mutation in Sequenzabschnitt"*//*"zweite Aminosäure-Mutation in Sequenzabschnitt",* ausgewählt aus der Gruppe bestehend aus: A//A, A//B, A//C, A//D, A//E, A//F, A//G; B//B, B//C, B//D, B//E, B//F, B//G; C//C, C//D, C//E, C//F, C//G; D//D, D//E, D//F, D//G; E//E, E//F, E//G; F//F, F//G; und G//G. Dabei bedeutet beispielsweise "E//F", dass die erste der wenigstens zwei Aminosäure-Mutationen im Sequenzabschnitt E) liegt und die zweite der wenigstens zwei Aminosäure-Mutationen im Sequenzabschnitt F) liegt.

In offenbarten Formen umfasst die Saccharose-Phosphorylase im Vergleich zu SEQ ID NO:1 wenigstens zwei Aminosäure-Mutationen, nämlich *"erste Aminosäure-Mutation in Sequenzabschnitt"*//*"zweite Aminosäure-Mutation in Sequenzabschnitt",* ausgewählt aus der Gruppe bestehend aus: A//C, A//D, A//G; B//E, B//G; C//D, C//F, C//G; D//F, D//G; E//G; und F//G.

In offenbarten Ausführungsformen der Saccharose-Phosphorylase ist die wenigstens eine Aminosäure-Mutation bzw. sind die wenigstens zwei, wenigstens drei oder wenigstens vier Aminosäure-Mutationen ausgewählt aus der Gruppe bestehend aus
- S21;
- H142, L151;
- H185;
- I295;
- N396;
- S451;
- D474, und T476.

Besonders bevorzugte erfindungsgemäße Saccharose-Phosphorylase umfassen wenigstens drei Aminosäure-Mutationen ausgewählt aus der Gruppe bestehend aus H185, I295 und D474.

Eine besonders bevorzugte erfindungsgemäße Saccharose-Phosphorylase umfasst eine oder zwei Aminosäure-Mutationen ausgewählt aus der Gruppe bestehend aus N396 und D474.

Eine besonders bevorzugte erfindungsgemäße Saccharose-Phosphorylase umfasst eine, zwei oder drei Aminosäure-Mutationen ausgewählt aus der Gruppe bestehend aus H142, N396 und D474.

Eine besonders bevorzugte erfindungsgemäße Saccharose-Phosphorylase umfasst eine, zwei oder drei Aminosäure-Mutationen ausgewählt aus der Gruppe bestehend aus L151, N396 und D474.

Eine besonders bevorzugte erfindungsgemäße Saccharose-Phosphorylase umfasst drei Aminosäure-Mutationen ausgewählt aus der Gruppe bestehend aus H185, I295 und T476.

Eine besonders bevorzugte erfindungsgemäße Saccharose-Phosphorylase umfasst drei Aminosäure-Mutationen ausgewählt aus der Gruppe bestehend aus H185, I295 und D474.

Eine offenbarte Saccharose-Phosphorylase umfasst eine, zwei, drei oder vier Aminosäure-Mutationen ausgewählt aus der Gruppe bestehend aus S21, H185, I295 und D474.

Eine offenbarte Saccharose-Phosphorylase umfasst eine, zwei, drei oder vier Aminosäure-Mutationen ausgewählt aus der Gruppe bestehend aus H185, I295, S451 und D474.

Bevorzugt weist die erfindungsgemäße Saccharose-Phosphorylase im Vergleich zu SEQ ID NO:1 bestimmte Aminosäure-Mutationen nicht aus. So ist es erfindungsgemäß bevorzugt, dass die erfindungsgemäße Saccharose-Phosphorylase wenigstens eine Aminosäure-Mutation ausgewählt aus der Gruppe bestehend aus Q331E, R393N, D445P, D446G, D446T, Q460E und E485H nicht umfasst, bevorzugt alle diese Aminosäure-Mutationen nicht umfasst.

In besonders bevorzugten Ausführungsformen der erfindungsgemäßen Saccharose-Phosphorylase ist die wenigstens eine Aminosäure-Mutation bzw. sind die wenigstens zwei, wenigstens drei oder wenigstens vier Aminosäure-Mutationen ausgewählt aus der Gruppe bestehend aus
- S21G;
- H142A, L151Y;
- H185G;
- I295V;
- N396S;
- S451T;
- D474E, und T476A.

Besonders bevorzugte erfindungsgemäße Saccharose-Phosphorylasen umfassen wenigstens eine, wenigstens zwei oder wenigstens drei Aminosäure-Mutationen ausgewählt aus der Gruppe bestehend aus H185G, I295V und D474E.

Eine besonders bevorzugte erfindungsgemäße Saccharose-Phosphorylase umfasst eine oder zwei Aminosäure-Mutationen ausgewählt aus der Gruppe bestehend aus N396S und D474E.

Eine besonders bevorzugte erfindungsgemäße Saccharose-Phosphorylase umfasst eine, zwei oder drei Aminosäure-Mutationen ausgewählt aus der Gruppe bestehend aus H142A, N396S und D474E.

Eine besonders bevorzugte erfindungsgemäße Saccharose-Phosphorylase umfasst eine, zwei oder drei Aminosäure-Mutationen ausgewählt aus der Gruppe bestehend aus L151Y, N396S und D474E.

Eine besonders bevorzugte erfindungsgemäße Saccharose-Phosphorylase umfasst eine, zwei oder drei Aminosäure-Mutationen ausgewählt aus der Gruppe bestehend aus H185G, I295V und T476A.

Eine besonders bevorzugte erfindungsgemäße Saccharose-Phosphorylase umfasst eine, zwei oder drei Aminosäure-Mutationen ausgewählt aus der Gruppe bestehend aus H185G, I295V und D474E.

Eine besonders bevorzugte erfindungsgemäße Saccharose-Phosphorylase umfasst eine, zwei, drei oder vier Aminosäure-Mutationen ausgewählt aus der Gruppe bestehend aus S21G, H185G, I295V und D474E.

Eine besonders bevorzugte erfindungsgemäße Saccharose-Phosphorylase umfasst eine, zwei, drei oder vier Aminosäure-Mutationen ausgewählt aus der Gruppe bestehend aus H185G, I295V, S451T und D474E.

Besonders bevorzugt umfasst die erfindungsgemäße Saccharose-Phosphorylase eine Aminosäure-Sequenz ausgewählt aus der Gruppe bestehend aus SEQ ID NO: 2, 3, 4, 5, 6, 7 und 8.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:2 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: H142, N396 und D474. Bevorzugt weist diese Aminosäure-Sequenz im Vergleich zu SEQ ID NO:1 eine oder mehrere der folgenden Aminosäure-Mutationen auf, vorzugsweise alle davon: H142A, N396S und D474E.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:3 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: L151, N396 und D474. Bevorzugt weist diese Aminosäure-Sequenz im Vergleich zu SEQ ID NO:1 eine oder mehrere der folgenden Aminosäure-Mutationen auf, vorzugsweise alle davon: L151Y, N396S und D474E.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:4 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 alle Aminosäure-Mutationen in folgenden Positionen auf: S21, H185, I295 und D474. Bevorzugt weist diese Aminosäure-Sequenz im Vergleich zu SEQ ID NO:1 eine oder mehrere der folgenden Aminosäure-Mutationen auf, vorzugsweise alle davon: S21G, H185G, I295V und D474E.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:5 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 alle Aminosäure-Mutationen in folgenden Positionen auf: H185, I295, S451 und D474. Bevorzugt weist diese Aminosäure-Sequenz im Vergleich zu SEQ ID NO:1 eine oder mehrere der folgenden Aminosäure-Mutationen auf, vorzugsweise alle davon: H185G, I295V, S451T und D474E.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:6 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 alle Aminosäure-Mutationen in folgenden Positionen auf: H185, I295 und T476. Bevorzugt weist diese Aminosäure-Sequenz im Vergleich zu SEQ ID NO:1 eine oder mehrere der folgenden Aminosäure-Mutationen auf, vorzugsweise alle davon: H185G, I295V und T476A.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:7 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: N396 und D474. Bevorzugt weist diese Aminosäure-Sequenz im Vergleich zu SEQ ID NO:1 eine oder mehrere der folgenden Aminosäure-Mutationen auf, vorzugsweise alle davon: N396S und D474E.

In einer bevorzugten Ausführungsform umfasst die erfindungsgemäße Saccharose-Phosphorylase eine Aminosäure-Sequenz, welche sich von SEQ ID NO:1 unterscheidet und welche eine Identität zur Aminosäuresequenz gemäß SEQ ID NO:8 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 alle Aminosäure-Mutationen in folgenden Positionen auf: H185, I295 und D474. Bevorzugt weist diese Aminosäure-Sequenz im Vergleich zu SEQ ID NO:1 eine oder mehrere der folgenden Aminosäure-Mutationen auf, vorzugsweise alle davon: H185G, I295V und D474E.

Typischerweise katalysiert die erfindungsgemäße Saccharose-Phosphorylase die Umsetzung von Saccharose und Phosphat zu Glucose-1-Phosphat und Fructose. Dabei weist die erfindungsgemäße Saccharose-Phosphorylase im Vergleich zur Saccharose-Phosphorylase gemäß SEQ ID NO: 1
(i) im Hinblick auf die Umsetzung von Saccharose und Phosphat zu Glucose-1-Phosphat und Fructose in Gegenwart äquimolarer Mengen Glucose-1-Phosphat und Fructose eine gesteigerte Aktivität; und/oder
(ii) im Hinblick auf die Umsetzung von Saccharose und Phosphat zu Glucose-1-Phosphat und Fructose in Gegenwart äquimolarer Mengen Glucose-1-Phosphat und Fructose eine geringere Hemmung durch Fructose; und/oder
(iii) eine höhere Temperaturstabilität nach Inkubation bei 58 °C für 15 min
   auf.

In nachfolgender Tabelle sind die Unterschiede dieser erfindungsgemäßen AminosäureSequenzen im Vergleich zur Saccharose-Phosphorylase aus *Bifidobacterium adolescentis* (Wildtyp (WT), SEQ ID NO:1) einander gegenübergestellt:

| SEQ ID NO: | Anzahl Mutationen | A | B | | C | D | E | F | G | |
|---|---|---|---|---|---|---|---|---|---|---|
| | | S21 | H142 | L151 | H185 | I295 | N396 | S451 | D474 | T476 |
| 1 | 0 | | | | | | | | | |
| 2 | 3 | | A | | | | S | | E | |
| 3 | 3 | | | Y | | | S | | E | |
| 4 | 4 | G | | | G | V | | | E | |
| 5 | 4 | | | | G | V | | T | E | |
| 6 | 3 | | | | G | V | | | | A |
| 7 | 2 | | | | | | S | | E | |
| 8 | 3 | | | | G | V | | | E | |

Ein weiterer Aspekt der Erfindung betrifft die Verwendung der vorstehend beschriebenen, erfindungsgemäßen Saccharose-Phosphorylase zur enzymatisch katalysierten Umsetzung von Saccharose und Phosphat zu Glucose-1-Phosphat und Fructose. Bevorzugt umfasst diese erfindungsgemäße Verwendung anschließend die Umsetzung des so erhaltenen Glucose-1-Phosphats mit Glucose zu Cellobiose unter enzymatischer Katalyse durch eine Cellobiose-Phosphorylase.

Alle bevorzugten Ausführungsformen, welche vorstehend im Zusammenhang mit der erfindungsgemäßen Saccharose-Phosphorylase beschrieben wurden, gelten entsprechend auch für ihre erfindungsgemäße Verwendung und werden daher an dieser Stelle nicht wiederholt.

Ein weiterer Aspekt der Erfindung betrifft ein Verfahren zur Herstellung von Glucose-1-Phosphat und Fructose umfassend die Umsetzung von Saccharose und Phosphat unter enzymatischer Katalyse durch die vorstehend beschriebene, erfindungsgemäße Saccharose-Phosphorylase.

Alle bevorzugten Ausführungsformen, welche vorstehend im Zusammenhang mit der erfindungsgemäßen Saccharose-Phosphorylase bzw. mit deren erfindungsgemäßer Verwendung beschrieben wurden, geltend entsprechend auch für das erfindungsgemäße Verfahren und werden daher an dieser Stelle nicht wiederholt.

Ein weiterer Aspekt der Erfindung betrifft ein Verfahren zur Herstellung von Cellobiose und Fructose aus Saccharose und Glucose, umfassend die Schritte
(a) Synthese von Glucose-1-Phosphat und Fructose durch Umsetzen von Saccharose und Phosphat unter enzymatischer Katalyse durch die vorstehend beschriebene, erfindungsgemäße Saccharose-Phosphorylase;
(b) Synthese von Cellobiose und Phosphat durch Umsetzen des Glucose-1-Phosphats mit Glucose unter enzymatischer Katalyse durch eine Cellobiose-Phosphorylase.

Alle bevorzugten Ausführungsformen, welche vorstehend im Zusammenhang mit der erfindungsgemäßen Saccharose-Phosphorylase bzw. mit deren erfindungsgemäßer Verwendung beschrieben wurden, geltend entsprechend auch für das erfindungsgemäße Verfahren und werden daher an dieser Stelle nicht wiederholt.

Weitere beschriebene Ausführungsformen sind:
Ausführungsform 1: Saccharose-Phosphorylase umfassend eine Aminosäuresequenz, welche eine Identität zur Aminosäuresequenz gemäß SEQ ID NO:1 von mindestens 80% aufweist und welche im Vergleich zu SEQ ID NO:1 wenigstens eine Aminosäure-Mutation umfasst
   - in einem Sequenzabschnitt A), welcher Positionen 11 bis 31 gemäß SEQ ID NO:1 entspricht; und/oder
   - in einem Sequenzabschnitt B), welcher Positionen 132 bis 161 gemäß SEQ ID NO:1 entspricht; und/oder
   - in einem Sequenzabschnitt C), welcher Positionen 175 bis 195 gemäß SEQ ID NO:1 entspricht; und/oder
   - in einem Sequenzabschnitt D), welcher Positionen 285 bis 305 gemäß SEQ ID NO:1 entspricht; und/oder
   - in einem Sequenzabschnitt E), welcher Positionen 394 bis 406 gemäß SEQ ID NO:1 entspricht; und/oder
   - in einem Sequenzabschnitt F), welcher Positionen 447 bis 459 gemäß SEQ ID NO:1 entspricht; und/oder
   - in einem Sequenzabschnitt G), welcher Positionen 464 bis 484 gemäß SEQ ID NO:1 entspricht.
Ausführungsform 2: Die Saccharose-Phosphorylase nach Ausführungsform 1, welche eine Identität zur Aminosäuresequenz gemäß SEQ ID NO:1 von mindestens 80% aufweist und welche im Vergleich zu SEQ ID NO:1 wenigstens eine Aminosäure-Mutation umfasst
   - in einem Sequenzabschnitt A), welcher Positionen 11 bis 31 gemäß SEQ ID NO:1 entspricht; und/oder
   - in einem Sequenzabschnitt C), welcher Positionen 175 bis 195 gemäß SEQ ID NO:1 entspricht; und/oder
   - in einem Sequenzabschnitt D), welcher Positionen 285 bis 305 gemäß SEQ ID NO:1 entspricht; und/oder
   - in einem Sequenzabschnitt G), welcher Positionen 464 bis 484 gemäß SEQ ID NO:1 entspricht.
Ausführungsform 3: Die Saccharose-Phosphorylase nach Ausführungsformen 1 oder 2, wobei
   - Sequenzabschnitt A) Positionen 16 bis 26 gemäß SEQ ID NO:1 entspricht; und/oder
   - Sequenzabschnitt B) Positionen 137 bis 156 gemäß SEQ ID NO:1 entspricht; und/oder
   - Sequenzabschnitt C) Positionen 180 bis 190 gemäß SEQ ID NO:1 entspricht; und/oder
   - Sequenzabschnitt D) Positionen 290 bis 300 gemäß SEQ ID NO:1 entspricht; und/oder
   - Sequenzabschnitt E) Positionen 394 bis 401 gemäß SEQ ID NO:1 entspricht; und/oder
   - Sequenzabschnitt F) Positionen 447 bis 456 gemäß SEQ ID NO:1 entspricht; und/oder
   - Sequenzabschnitt G) Positionen 469 bis 481 gemäß SEQ ID NO:1 entspricht.
Ausführungsform 4: Die Saccharose-Phosphorylase nach einer der vorstehenden Ausführungsformen, wobei
   - Sequenzabschnitt A) Positionen 20 bis 22 gemäß SEQ ID NO:1 entspricht; und/oder
   - Sequenzabschnitt B) Positionen 141 bis 152 gemäß SEQ ID NO:1 entspricht; und/oder
   - Sequenzabschnitt C) Positionen 184 bis 186 gemäß SEQ ID NO:1 entspricht; und/oder
   - Sequenzabschnitt D) Positionen 294 bis 296 gemäß SEQ ID NO:1 entspricht; und/oder
   - Sequenzabschnitt E) Positionen 395 bis 397 gemäß SEQ ID NO:1 entspricht; und/oder
   - Sequenzabschnitt F) Positionen 450 bis 452 gemäß SEQ ID NO:1 entspricht; und/oder
   - Sequenzabschnitt G) Positionen 473 bis 477 gemäß SEQ ID NO:1 entspricht.
Ausfuhrungsform 5: Die Saccharose-Phosphorylase nach einer der vorstehenden Ausführungsformen, wobei die wenigstens eine Aminosäure-Mutation ausgewählt ist aus der Gruppe bestehend aus
   - S21;
   - H142, L151;
   - H185;
   - I295;
   - N396;
   - S451;
   - D474, und T476.
Ausführungsform 6: Die Saccharose-Phosphorylase nach einer der vorstehenden Ausführungsformen, welche wenigstens zwei, drei oder vier Aminosäure-Mutationen umfasst, welche jeweils unabhängig voneinander definiert sind wie in Anspruch 1 oder 2.
Ausführungsform 7: Die Saccharose-Phosphorylase nach einer der vorstehenden Ausführungsformen, welche wenigstens eine Aminosäure-Mutation ausgewählt aus der Gruppe bestehend aus Q331E, R393N, D445P, D446G, D446T, Q460E und E485H nicht umfasst, bevorzugt alle diese Aminosäure-Mutationen nicht umfasst.
Ausführungsform 8: Die Saccharose-Phosphorylase nach einer der vorstehenden Ausführungsformen, wobei die wenigstens eine Aminosäure-Mutation ausgewählt ist aus der Gruppe bestehend aus
   - S21G;
   - H142A, L151Y;
   - H185G;
   - I295V;
   - N396S;
   - S451T;
   - D474E, und T476A.
Ausführungsform 9: Die Saccharose-Phosphorylase nach einer der vorstehenden Ausführungsformen, welche die Umsetzung von Saccharose und Phosphat zu Glucose-1-Phosphat und Fructose katalysiert.
Ausführungsform 10: Die Saccharose-Phosphorylase nach einer der vorstehenden Ausführungsformen, welche im Vergleich zur Saccharose-Phosphorylase gemäß SEQ ID NO:1
   (i) im Hinblick auf die Umsetzung von Saccharose und Phosphat zu Glucose-1-Phosphat und Fructose in Gegenwart äquimolarer Mengen Glucose-1-Phosphat und Fructose eine gesteigerte Aktivität; und/oder
   (ii) im Hinblick auf die Umsetzung von Saccharose und Phosphat zu Glucose-1-Phosphat und Fructose in Gegenwart äquimolarer Mengen Glucose-1-Phosphat und Fructose eine geringere Hemmung durch Fructose; und/oder
   (iii) eine höhere Temperaturstabilität nach Inkubation bei 58 °C für 15 min aufweist.
Ausführungsform 11: Die Saccharose-Phosphorylase nach einer der vorstehenden Ausführungsformen, welche eine Identität zur Aminosäuresequenz gemäß SEQ ID NO:4 von mindestens 90% aufweist.
Ausführungsform 12: Die Saccharose-Phosphorylase nach Ausführungsform 11, wobei die Identität zur Aminosäuresequenz gemäß SEQ ID NO:4 mindestens 95% beträgt.
Ausführungsform 13: Verwendung der Saccharose-Phosphorylase nach einer der Ausführungsformen 1 bis 12 zur enzymatisch katalysierten Umsetzung von Saccharose und Phosphat zu Glucose-1-Phosphat und Fructose.
Ausführungsform 14: Ein Verfahren zur Herstellung von Glucose-1-Phosphat und Fructose umfassend die Umsetzung von Saccharose und Phosphat unter enzymatischer Katalyse durch die Saccharose-Phosphorylase nach einer der Ausführungsformen 1 bis 12.
Ausführungsform 15: Ein Verfahren zur Herstellung von Cellobiose und Fructose aus Saccharose und Glucose, umfassend die Schritte
   (a) Synthese von Glucose-1-Phosphat und Fructose durch Umsetzen von Saccharose und Phosphat unter enzymatischer Katalyse durch die Saccharose-Phosphorylase nach einer der Ausführungsformen 1 bis 12;
   (b) Synthese von Cellobiose und Phosphat durch Umsetzen des Glucose-1-Phsophats mit Glucose unter enzymatischer Katalyse durch eine Cellobiose-Phosphorylase.

Darüber hinaus sind weitere beschriebene Ausführungsformen:
Ausführungsform 16: Saccharose-Phosphorylase umfassend eine Aminosäuresequenz, welche eine Identität zur Aminosäuresequenz gemäß SEQ ID NO:1 von mindestens 80% aufweist und welche im Vergleich zu SEQ ID NO:1 wenigstens zwei Aminosäure-Mutation umfasst
   - in einem Sequenzabschnitt B), welcher Positionen 132 bis 161 gemäß SEQ ID NO:1 entspricht; und/oder
   - in einem Sequenzabschnitt E), welcher Positionen 394 bis 406 gemäß SEQ ID NO:1 entspricht; und/oder
   - in einem Sequenzabschnitt G), welcher Positionen 464 bis 485 gemäß SEQ ID NO:1 entspricht.
Ausführungsform 17: Die Saccharose-Phosphorylase nach Ausführungsform 16, wobei
   - Sequenzabschnitt B) Positionen 137 bis 156 gemäß SEQ ID NO:1 entspricht; und/oder
   - Sequenzabschnitt E) Positionen 394 bis 401 gemäß SEQ ID NO:1 entspricht; und/oder
   - Sequenzabschnitt G) Positionen 469 bis 481 gemäß SEQ ID NO:1 entspricht.
Ausführungsform 18: Die Saccharose-Phosphorylase nach Ausführungsform 17, wobei
   - Sequenzabschnitt B) Positionen 141 bis 152 gemäß SEQ ID NO:1 entspricht; und/oder
   - Sequenzabschnitt E) Positionen 395 bis 397 gemäß SEQ ID NO:1 entspricht; und/oder
   - Sequenzabschnitt G) Positionen 473 bis 477 gemäß SEQ ID NO:1 entspricht.
Ausführungsform 19: Die Saccharose-Phosphorylase nach Ausführungsform 18, wobei die Aminosäure-Mutation
   - H142 oder L151, und/oder
   - N396, und/oder
   - D474 oder T476 ist.
Ausführungsform 20: Die Saccharose-Phosphorylase nach Ausführungsform 19, wobei die Aminosäure-Mutation
   - H142 und/oder
   - N396, und/oder
   - D474 ist.
Ausführungsform 21: Die Saccharose-Phosphorylase nach Ausführungsform 19, wobei die Aminosäure-Mutation
   - L151, und/oder
   - N396, und/oder
   - D474 ist.
Ausführungsform 22: Die Saccharose-Phosphorylase nach einer der Ausführungsformen 19 bis 21, wobei die Aminosäure-Mutationen
   - H142A oder L151Y, und/oder
   - N396S, und/oder
   - D474E oder T476A sind.

Die nachfolgenden Beispiele illustrieren die Erfindung, sind jedoch nicht einschränkend auszulegen:

### Beispiel 1 - Engineering der Wildtyp-Saccharose-Phosphorylase von Bifidobacterium adolescentis

Für das Engineering des Wildtyps der Saccharose-Phosphorylase von *Bifidobacterium adolescentis* wurden folgende Engineering-Ziele festgelegt:
- Reduktion der Fructose-Hemmung
- Erhöhung der Aktivität allgemein
- Reduktion des Km-Wert bzgl. Phosphat
- Reduktion der G1P-Hemmung
- Erhaltung der Temperaturstabilität

Für das Engineering wurde ein semirationaler, iterativer Ansatz gewählt. In der ersten Runde Engineering wurden zunächst nach Analyse von allen verfügbaren Daten potentiell interessante Positionen und mögliche Austausche identifiziert und als Einzelmutanten erstellt. Diese wurden dann in einem Multiparameter-Screening nach den gewünschten Engineering-Zielen gescreent. Positive Mutationen aus dieser ersten Runde wurden dann im Folgenden kombiniert und die sich daraus ergebenden Varianten charakterisiert.

Alle Assays wurden bei 30 °C und pH 6,3 durchgeführt. Als G1P wurde ausschließlich kommerzielles G1P (Sigma-Aldrich, Artikel-Nr. G7000) verwendet. Als Vergleichsenzym wurde immer das Wildtypenzym (mit neuer Codon-Optimierung) zweimal auf jeder Platte mitgeführt. Alle Daten wurden auf ein Vergleichsenzym, welches auf derselben Platte mitgeführt wurde, normiert, wodurch Schwankungen der Expression oder Messungen zwischen verschiedenen Platten ausgeglichen werden konnten.

**Übersicht Multiparameterscreening:**

| Engineering-Ziel | Versuchsbedingungen | Detektion |
|---|---|---|
| Steigerung der Phosphorolyse-Aktivität | 750 mM Saccharose + 750 mM Pi | ΔG1P |
| Erniedrigung der Fructose-Hemmung | 750 mM Saccharose + 750 mM Pi + 250 mM Fructose | ΔG1P |
| Erniedrigung des Km-Wert Pi | 750 mM Saccharose + 20 mM Pi | ΔG1P |
| Erhaltung der Temperaturstabilität | Vorbehandlung 15 min/65°C; Reaktion mit 750 mM Saccharose + 750 mM Pi | ΔG1P |

Durch MDM-Analyse wurden für eine erste Runde Engineering 169 Positionen und Austausche ausgewählt. Dies entspricht etwa 33% aller Positionen aber weniger als 2% aller möglichen Einzelmutanten. Diese Einzelmutanten wurden mit der AGM-Methode (*automated generation of mutants*) erstellt. Als Template wurde die Sequenz des Wildtyp Gens verwendet (mit Codon-Optimierung). Die erstellten Varianten wurden exprimiert und nach den unterschiedlichen Engineering-Zielen gescreent. Die Ergebnisse wurden auf den zweifach auf jeder Platte mitgeführten Wildtyp normiert. Es konnten für alle Engineering-Ziele verbesserte Varianten identifiziert werden.

In einer zweiten Runde Engineering wurden die 7 besten Mutationen aus der ersten Runde miteinander kombiniert (128 Varianten). Die Erstellung der Komplexbank erfolgte mit der Mutagenese Methode gemäß WO2009/146892. 376 Klone der Komplexbank wurden gepickt, exprimiert und in Bezug auf die Engineering-Ziele gescreent (2,9-faches Oversampling, Screeningabdeckung 95%). Es konnten für alle Engineering-Ziele verbesserte Varianten identifiziert werden.

In einer dritten Runde Engineering wurden die Hits aus der zweiten Runde noch mit weiteren Primärhits aus der 1. Runde kombiniert (105 Varianten). Diese Varianten wurden mit der AGM-Methode (*automated generation ofmutants*) erstellt, exprimiert und gescreent.

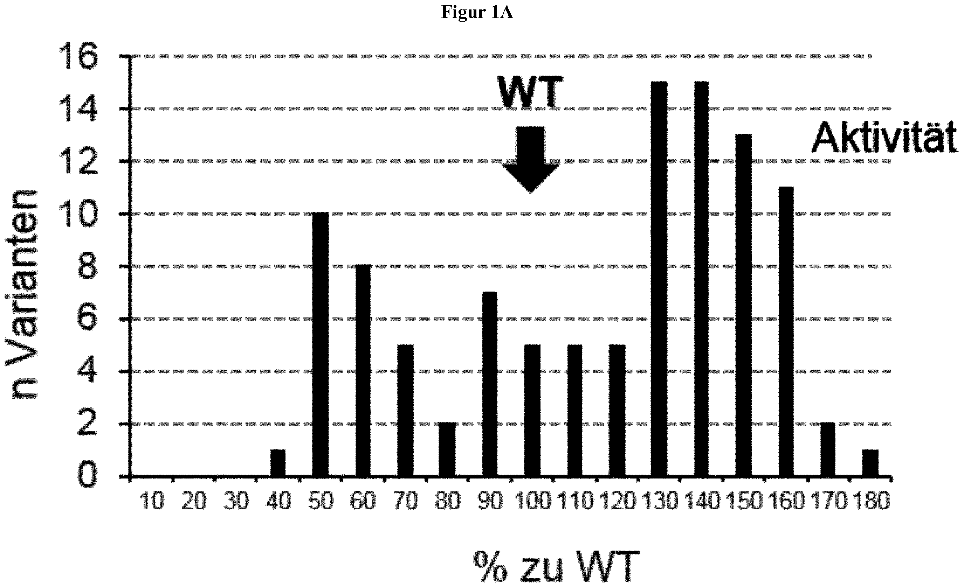

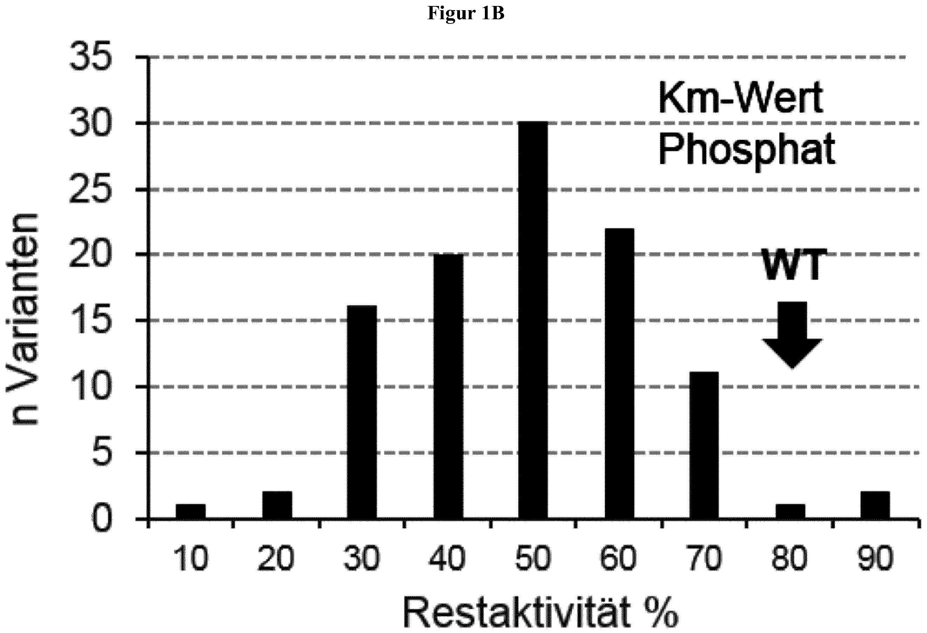

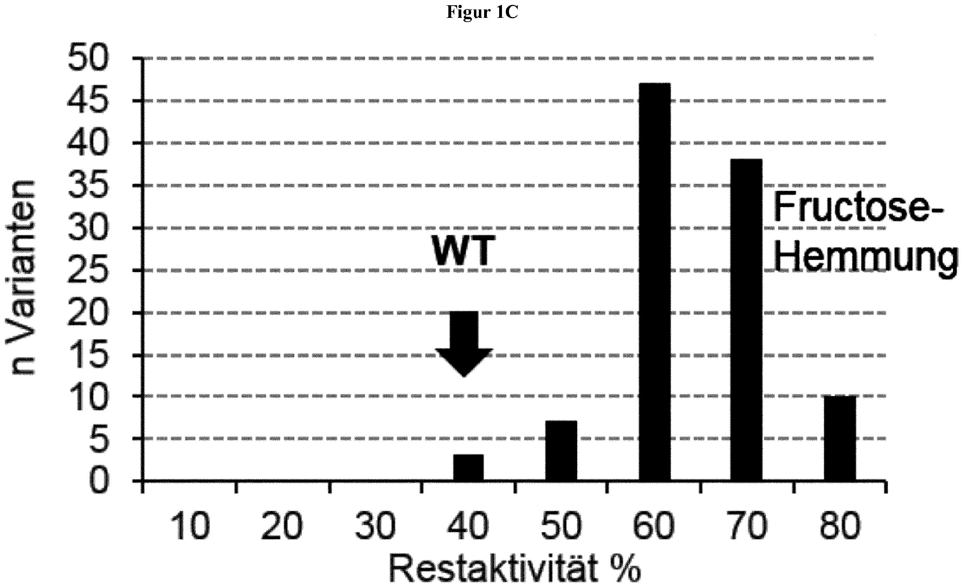

Die Ergebnisse des Engineerings hinsichtlich Phosphorolyse-Aktivität, Fructose-Hemmung sowie Km-Wert bezüglich Phosphat (Pi) sind vergleichend zum Wildtyp (WT) in Figuren 1A bis 1C dargestellt.

### Beispiel 2 - Charakterisierung der 7 besten Varianten:

Die besten 7 Varianten aus der zweiten und dritten Runde Engineering wurden für eine detaillierte, finale Charakterisierung ausgewählt (SEQ ID NO:2 bis 8):

| SEQ ID NO: | Referenz | | A | B | | C | D | E | F | G | |
|---|---|---|---|---|---|---|---|---|---|---|---|
| | | | S21 | H142 | L151 | H185 | I295 | N396 | S451 | D474 | T476 |
| 1 | WT | pSE014 | | | | | | | | | |
| 2 | P1-C7 | pPB115 | | A | | | | S | | E | |
| 3 | P1-C10 | pPB 116 | | | Y | | | S | | E | |
| 4 | P2-A3 | pPB 117 | G | | | G | V | | | E | |
| 5 | P2-B7 | pPB 118 | | | | G | V | | T | E | |
| 6 | P2-B9 | pPB 119 | | | | G | V | | | | A |
| 7 | PB 10-24-1 | pPB111 | | | | | | S | | E | |
| 8 | PB 10-24-3 | pPB113 | | | | G | V | | | E | |

### a) Aktivitätsausbeute und lösliche Expression

Die ausgewählten sieben Varianten sowie das Wildtyp-Enzym (WT) wurden in zwei unabhängigen Expressionskulturen im Schüttelkolben exprimiert, aufgeschlossen und die Aktivitätsausbeute pro mL Expressionskultur bestimmt:

| SEQ ID NO: | Plasmid ID | PU/mL Kultur | ± s | Faktor WT |
|---|---|---|---|---|
| 1 (= WT) | pSE014 | 35,4 | ± 1,1 | 1,00 |
| 2 | pPB115 | 35,6 | ± 2,2 | 1,00 |
| 3 | pPB 116 | 46,1 | ± 1,7 | 1,30 |
| 4 | pPB 117 | 47,1 | ± 3,0 | 1,33 |
| 5 | pPB118 | 34,9 | ± 0,3 | 0,99 |
| 6 | pPB 119 | 40,7 | ± 1,9 | 1,15 |
| 7 | pPB111 | 46,3 | ± 2,4 | 1,31 |
| 8 | pPB113 | 41,2 | ± 2,5 | 1,16 |

Zur Beurteilung der löslichen Expression wurde die lösliche Fraktion normiert. Es wurden keine signifikanten Unterschiede in der Expressionsstärke zwischen den unterschiedlichen Varianten beobachtet.

### b) Schmelzkurven

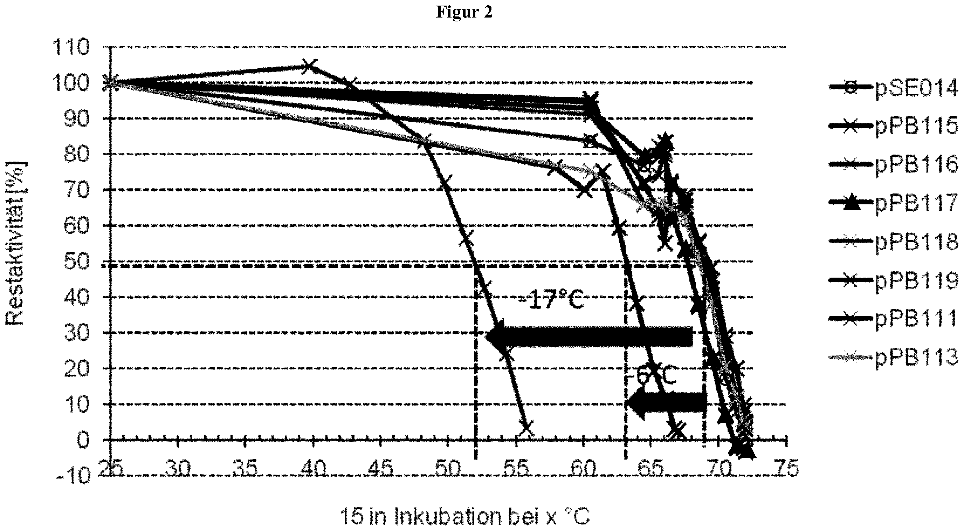

Eines der Ziele des Engineerings war, die hohe Temperaturstabilität der Saccharose-Phosphorylase des Wildtyps zu erhalten. Zur Prüfung der Varianten wurden die Enzyme für 15 min bei Temperaturen zwischen 40 und 72 °C im PCR-Cycler inkubiert und anschließend die Rest-Aktivität der Enzyme bestimmt. Fünf der untersuchten Varianten zeigten die gleiche Temperaturstabilität wie das Wildtyp-Enzym. Die Ergebnisse sind in Figur 2 dargestellt.

### c) Fructose-Hemmung, GIP-Hemmung und Km-Wert Phosphat

Um mögliche Veränderungen der Varianten bezügliche der Fructose- bzw. G1P-Hemmung zu beurteilen, wurden für die Enzymvarianten sowie für das Wildtyp-Enzym jeweils die Veränderungen der initialen Aktivitäten in Gegenwart steigender Fructose- bzw. G1P-Konzentrationen gemessen. Als Substrat wurden jeweils 200 mM Saccharose und Phosphat zugegeben.

Es zeigte sich, dass alle untersuchten Varianten eine deutlich geringere Hemmung in Gegenwart von 250-750 mM Fructose aufwiesen. Auch wenn die Fructosehemmung nicht vollständig eliminiert werden konnte, ist der Grad der Verbesserung bemerkenswert. Für die G1P-Hemmung hingegen waren die beobachteten Verbesserungen nicht so deutlich. Allerdings ist auch die G1P-Hemmung des Wildtyps nicht so stark ausgeprägt, wie die Fructose-Hemmung. Zusätzlich kommt die G1P-Hemmung nur bei G1P-Konzentrationen über 100 mM zum Tragen.

Analog zu den Versuchen zur Enzym-Hemmung, wurde die initiale Aktivität in Gegenwart von 10-250 mM Phosphat für alle Varianten und das Wildtyp-Enzym bestimmt, um Veränderungen des Km-Wert bezüglich Phosphat zu erfassen. Keine der Varianten zeigte signifikante Unterschiede in der initialen Aktivität im Vergleich zum Wildtyp bei unterschiedlichen Phosphatkonzentrationen. Es zeigte keine Variante eine Verschlechterung gegenüber dem Wildtyp.

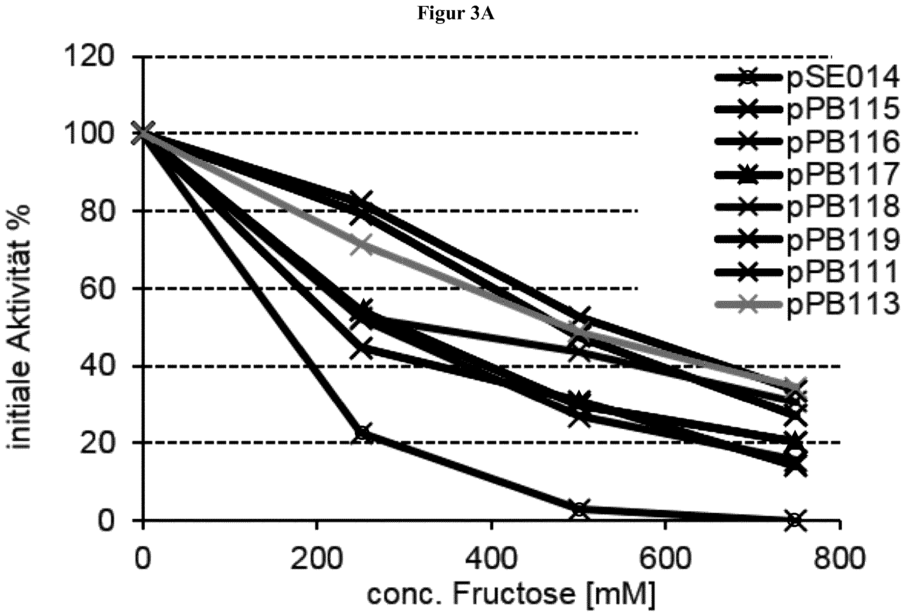

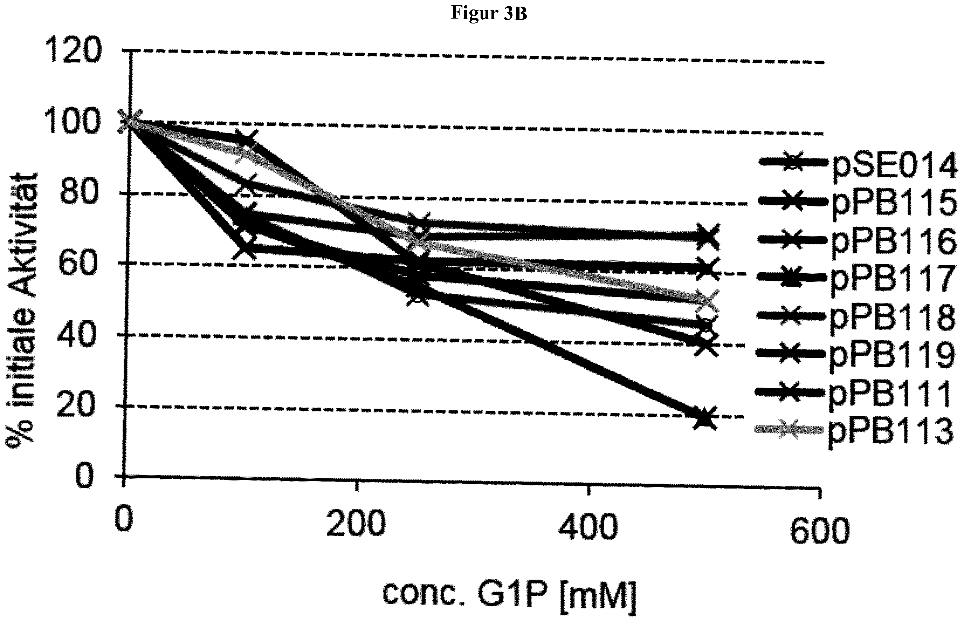

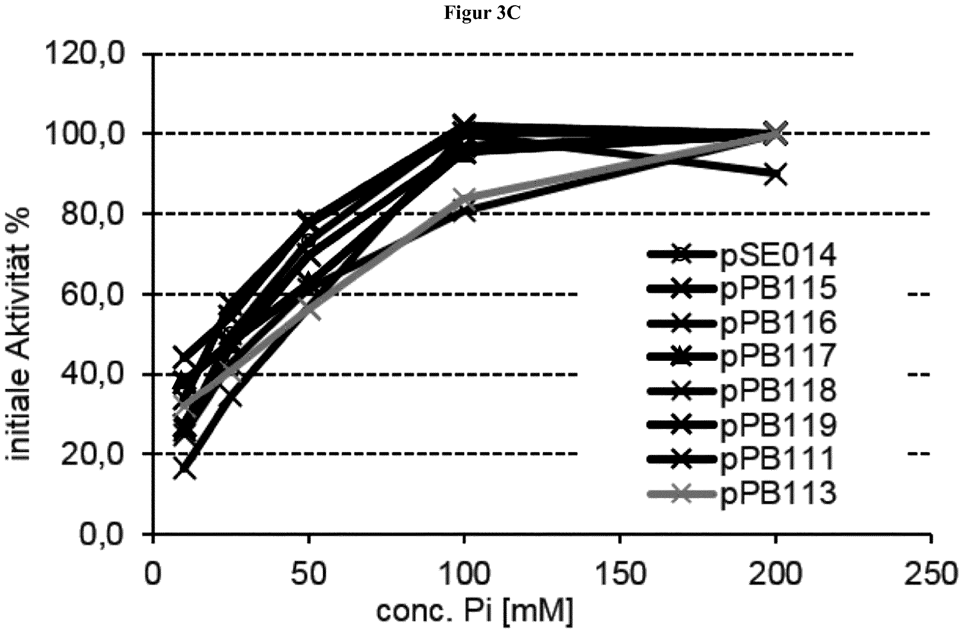

Die Ergebnisse hinsichtlich Fructose-, Phosphat- und Glucose-1-Phosphat-Hemmung sind in Figuren 3A bis 3C dargestellt.

Vier ausgewählte Varianten wurden im Schüttelkolben exprimiert, geerntet, in 50 mM Na-MES-Puffer pH 6,3 resuspendiert und mit Ultraschall aufgeschlossen. Zellfragmente und unlösliche Bestandteile wurden durch Zentrifugation abgetrennt. Der Überstand wurde steril-filtriert und 50/50 v/v mit sterilem Glycerin versetzt. Die Phosphorolyse-Aktivitäten der formulierten Enzyme wurden bestimmt:

| SEQ ID NO: | Referenz | Volumen [mL] | Phosphorolyse-Aktivität [PU/mL] |
|---|---|---|---|
| 8 | pPB113 | 37,5 | 408,5 |
| 4 | pPB 117 | 45 | 460,2 |
| 5 | pPB118 | 47 | 384,3 |
| 6 | pPB 119 | 43,5 | 444,1 |

## Patentansprüche

1. Saccharose-Phosphorylase umfassend eine Aminosäuresequenz, welche eine Identität zur Aminosäuresequenz gemäß SEQ ID NO:1 von mindestens 80% aufweist;
welche im Vergleich zu SEQ ID NO:1 eine Aminosäure-Mutation in Position H185 und eine Aminosäure-Mutation in Position I295 umfasst; und
welche im Vergleich zur Saccharose-Phosphorylase gemäß SEQ ID NO:1
(i) im Hinblick auf die Umsetzung von Saccharose und Phosphat zu Glucose-1-Phosphat und Fructose in Gegenwart äquimolarer Mengen Glucose-1-Phosphat und Fructose eine gesteigerte Aktivität; und/oder
(ii) im Hinblick auf die Umsetzung von Saccharose und Phosphat zu Glucose-1-Phosphat und Fructose in Gegenwart äquimolarer Mengen Glucose-1-Phosphat und Fructose eine geringere Hemmung durch Fructose; und/oder
(iii) eine höhere Temperaturstabilität nach Inkubation bei 58 °C für 15 min aufweist.

2. Die Saccharose-Phosphorylase nach Anspruch 1, wobei die Aminosäure-Mutationen H185G und I295V sind.

3. Die Saccharose-Phosphorylase nach Anspruch 1 oder 2, welche zusätzlich eine Aminosäure-Mutation in Position D474 oder Position T476 umfasst.

4. Die Saccharose-Phosphorylase nach Anspruch 3, wobei die Aminosäure-Mutation D474E oder T476A ist.

5. Die Saccharose-Phosphorylase nach einem der vorstehenden Ansprüche, welche zusätzlich eine Aminosäure-Mutation in Position S21 umfasst.

6. Die Saccharose-Phosphorylase nach Anspruch 5, wobei die Aminosäure-Mutation S21G ist.

7. Die Saccharose-Phosphorylase nach einem der vorstehenden Ansprüche, welche zusätzlich eine Aminosäure-Mutation in Position S451 umfasst.

8. Die Saccharose-Phosphorylase nach Anspruch 7, wobei die Aminosäure-Mutation S451T ist.

9. Die Saccharose-Phosphorylase nach einer der vorstehenden Ansprüche, welche wenigstens eine Aminosäure-Mutation ausgewählt aus der Gruppe bestehend aus Q331E, R393N, D445P, D446G, D446T, Q460E und E485H nicht umfasst, bevorzugt alle diese Aminosäure-Mutationen nicht umfasst.

10. Die Saccharose-Phosphorylase nach einem der vorstehenden Ansprüche, welche
(i) eine Identität zur Aminosäuresequenz gemäß SEQ ID NO:4 von mindestens 90% aufweist; bevorzugt mindestens 95%; oder
(ii) eine Identität zur Aminosäuresequenz gemäß SEQ ID NO:5 von mindestens 90% aufweist; bevorzugt mindestens 95% beträgt; oder
(iii) eine Identität zur Aminosäuresequenz gemäß SEQ ID NO:6 von mindestens 90% aufweist; bevorzugt mindestens 95%; oder
(iv) eine Identität zur Aminosäuresequenz gemäß SEQ ID NO:8 von mindestens 90% aufweist; bevorzugt mindestens 95%.

11. Verwendung der Saccharose-Phosphorylase nach einem der Ansprüche 1 bis 10 zur enzymatisch katalysierten Umsetzung von Saccharose und Phosphat zu Glucose-1-Phosphat und Fructose.

12. Ein Verfahren zur Herstellung von Glucose-1-Phosphat und Fructose umfassend die Umsetzung von Saccharose und Phosphat unter enzymatischer Katalyse durch die Saccharose-Phosphorylase nach einem der Ansprüche 1 bis 10.

13. Ein Verfahren zur Herstellung von Cellobiose und Fructose aus Saccharose und Glucose, umfassend die Schritte
(a) Synthese von Glucose-1-Phosphat und Fructose durch Umsetzen von Saccharose und Phosphat unter enzymatischer Katalyse durch die Saccharose-Phosphorylase nach einem der Ansprüche 1 bis 10;
(b) Synthese von Cellobiose und Phosphat durch Umsetzen des Glucose-1-Phsophats mit Glucose unter enzymatischer Katalyse durch eine Cellobiose-Phosphorylase.

## Claims

1. Sucrose phosphorylase comprising an amino acid sequence which has an identity in relation to the amino acid sequence according to SEQ ID NO:1 of at least 80%;
which, in comparison with SEQ ID NO:1, comprises an amino acid mutation in position H185 and an amino acid mutation in position 1295; and
which, in comparison with the sucrose phosphorylase according to SEQ ID NO:1, has
(i) an increased activity with respect to the conversion of sucrose and phosphate to glucose 1-phosphate and fructose in the presence of equimolar amounts of glucose 1-phosphate and fructose; and/or
(ii) a lower inhibition by fructose with respect to the conversion of sucrose and phosphate to glucose 1-phosphate and fructose in the presence of equimolar amounts of glucose 1-phosphate and fructose; and/or
(iii) a higher temperature stability after incubation at 58°C for 15 min.

2. Sucrose phosphorylase according to Claim 1, wherein the amino acid mutations are H185G and I295V.

3. Sucrose phosphorylase according to Claim 1 or 2, which additionally comprises an amino acid mutation in position D474 or position T476.

4. Sucrose phosphorylase according to Claim 3, wherein the amino acid mutation is D474E or T476A.

5. Sucrose phosphorylase according to any of the preceding claims, which additionally comprises an amino acid mutation in position S21.

6. Sucrose phosphorylase according to Claim 5, wherein the amino acid mutation is S21G.

7. Sucrose phosphorylase according to any of the preceding claims, which additionally comprises an amino acid mutation in position S451.

8. Sucrose phosphorylase according to Claim 7, wherein the amino acid mutation is S451T.

9. Sucrose phosphorylase according to any of the preceding claims, which does not comprise at least one amino acid mutation selected from the group consisting of Q331E, R393N, D445P, D446G, D446T, Q460E and E485H, preferably does not comprise all these amino acid mutations.

10. Sucrose phosphorylase according to any of the preceding claims, which
(i) has an identity in relation to the amino acid sequence according to SEQ ID NO:4 of at least 90%; preferably at least 95%; or
(ii) has an identity in relation to the amino acid sequence according to SEQ ID NO:5 of at least 90%; is preferably at least 95%; or
(iii) has an identity in relation to the amino acid sequence according to SEQ ID NO:6 of at least 90%; preferably at least 95%; or
(iv) has an identity in relation to the amino acid sequence according to SEQ ID NO:8 of at least 90%; preferably at least 95%.

11. Use of the sucrose phosphorylase according to any of Claims 1 to 10 for the enzymatically catalysed conversion of sucrose and phosphate to glucose 1-phosphate and fructose.

12. Method for producing glucose 1-phosphate and fructose, comprising the conversion of sucrose and phosphate under enzymatic catalysis by the sucrose phosphorylase according to any of Claims 1 to 10.

13. Method for producing cellobiose and fructose from sucrose and glucose, comprising the steps of
(a) synthesis of glucose 1-phosphate and fructose by conversion of sucrose and phosphate under enzymatic catalysis by the sucrose phosphorylase according to any of Claims 1 to 10;
(b) synthesis of cellobiose and phosphate by reaction of the glucose 1-phosphate with glucose under enzymatic catalysis by a cellobiose phosphorylase.

## Revendications

1. Saccharose-phosphorylase comprenant une séquence d'acides aminés, qui présente une identité avec la séquence d'acides aminés selon SEQ ID NO : 1 d'au moins 80 %;
qui comprend en comparaison de SEQ ID NO : 1 une mutation d'acides aminés en position H185 et une mutation d'acides aminés en position I295 ; et
qui présente en comparaison de la saccharose-phosphorylase selon SEQ ID NO : 1
(i) au regard de la transformation de saccharose et phosphate en glucose-1-phosphate et fructose en présence de quantités équimolaires de glucose-1-phosphate et fructose, une activité augmentée ; et/ou
(ii) au regard de la transformation de saccharose et phosphate en glucose-1-phosphate et fructose en présence de quantités équimolaires de glucose-1-phosphate et fructose, une inhibition plus faible par le fructose ; et/ou
(iii) une température de stabilité plus élevée après incubation à 58 °C pendant 15 minutes.

2. Saccharose-phosphorylase selon la revendication 1, dans laquelle les mutations d'acides aminés sont H185G et I295V.

3. Saccharose-phosphorylase selon la revendication 1 ou 2, qui comprend en outre une mutation d'acides aminés en position D474 ou en position T476.

4. Saccharose-phosphorylase selon la revendication 3, dans laquelle la mutation d'acides aminés est D474E ou T476A.

5. Saccharose-phosphorylase selon l'une quelconque des revendications précédentes, qui comprend en outre une mutation d'acides aminés en position S21.

6. Saccharose-phosphorylase selon la revendication 5, dans laquelle la mutation d'acides aminés est S21G.

7. Saccharose-phosphorylase selon l'une quelconque des revendications précédentes, qui comprend en outre une mutation d'acides aminés en position S451.

8. Saccharose-phosphorylase selon la revendication 7, dans laquelle la mutation d'acides aminés est S451T.

9. Saccharose-phosphorylase selon l'une quelconque des revendications précédentes, qui ne comprend pas au moins une mutation d'acides aminés choisie dans le groupe constitué par Q331E, R393N, D445P, D446G, D446T, Q460E et E485H, de préférence qui ne comprend pas toutes ces mutations d'acides aminés.

10. Saccharose-phosphorylase selon l'une quelconque des revendications précédentes, qui présente :
(i) une identité avec la séquence d'acides aminés selon SEQ ID NO : 4 d'au moins 90 % ; de préférence d'au moins 95 % ; ou
(ii) une identité avec la séquence d'acides aminés selon SEQ ID NO : 5 d'au moins 90 % ; est de préférence au moins 95 % ; ou
(iii) une identité avec la séquence d'acides aminés selon SEQ ID NO : 6 d'au moins 90 % ; de préférence d'au moins 95 % ; ou
(iv) une identité avec la séquence d'acides aminés selon SEQ ID NO : 8 d'au moins 90 % ; de préférence d'au moins 95 %.

11. Utilisation de la saccharose-phosphorylase selon l'une quelconque des revendications 1 à 10 pour la transformation sous catalyse enzymatique de saccharose et phosphate en glucose-1-phosphate et fructose.

12. Procédé de fabrication de glucose-1-phosphate et fructose comprenant la transformation de saccharose et phosphate sous catalyse enzymatique par la saccharose-phosphorylase selon l'une quelconque des revendications 1 à 10.

13. Procédé de fabrication de cellobiose et fructose à partir de saccharose et glucose, comprenant les étapes suivantes :
(a) la synthèse de glucose-1-phosphate et fructose par transformation de saccharose et phosphate sous catalyse enzymatique par la saccharose-phosphorylase selon l'une quelconque des revendications 1 à 10 ;
(b) la synthèse de cellobiose et phosphate par transformation du glucose-1-phosphate avec du glucose sous catalyse enzymatique par une cellobiose-phosphorylase.


### SEQUENCE LISTING

<110> Pfeifer & Langen GmbH & Co. KG
<120> Saccharose Phosphorylase
<130> PFL0013-WO
<150> EP16159122
   <151> 2016-03-08
<160> 8
<170> PatentIn version 3.5
<210> 1
   <211> 504
   <212> PRT
   <213> Bifidobacterium adolescentis
<400> 1
<210> 2
   <211> 504
   <212> PRT
   <213> artificial
<220>
   <223> modified sequence
<400> 2
   Met Lys Asn Lys Val Gln Leu Ile Thr Tyr Ala Asp Arg Leu Gly Asp
   1               5                   10                  15
   Gly Thr Ile Lys Ser Met Thr Asp Ile Leu Arg Thr Arg Phe Asp Gly
               20                  25                  30
   Val Tyr Asp Gly Val His Ile Leu Pro Phe Phe Thr Pro Phe Asp Gly
           35                  40                  45
   Ala Asp Ala Gly Phe Asp Pro Ile Asp His Thr Lys Val Asp Glu Arg
       50                  55                  60
   Leu Gly Ser Trp Asp Asp Val Ala Glu Leu Ser Lys Thr His Asn Ile
   65                  70                  75                  80
   Met Val Asp Ala Ile Val Asn His Met Ser Trp Glu Ser Lys Gln Phe
                   85                  90                  95
   Gln Asp Val Leu Ala Lys Gly Glu Glu Ser Glu Tyr Tyr Pro Met Phe
               100                 105                 110
   Leu Thr Met Ser Ser Val Phe Pro Asn Gly Ala Thr Glu Glu Asp Leu
   115 120 125
   Ala Gly Ile Tyr Arg Pro Arg Pro Gly Leu Pro Phe Thr Ala Tyr Lys
   130 135 140
   Phe Ala Gly Lys Thr Arg Leu Val Trp Val Ser Phe Thr Pro Gln Gln
   145 150 155 160
   Val Asp Ile Asp Thr Asp Ser Asp Lys Gly Trp Glu Tyr Leu Met Ser
   165 170 175
   Ile Phe Asp Gln Met Ala Ala Ser His Val Ser Tyr Ile Arg Leu Asp
   180 185 190
   Ala Val Gly Tyr Gly Ala Lys Glu Ala Gly Thr Ser Cys Phe Met Thr
   195 200 205
   Pro Lys Thr Phe Lys Leu Ile Ser Arg Leu Arg Glu Glu Gly Val Lys
   210 215 220
   Arg Gly Leu Glu Ile Leu Ile Glu Val His Ser Tyr Tyr Lys Lys Gln
   225 230 235 240
   Val Glu Ile Ala Ser Lys Val Asp Arg Val Tyr Asp Phe Ala Leu Pro
   245 250 255
   Pro Leu Leu Leu His Ala Leu Ser Thr Gly His Val Glu Pro Val Ala
   260 265 270
   His Trp Thr Asp Ile Arg Pro Asn Asn Ala Val Thr Val Leu Asp Thr
   275 280 285
   His Asp Gly Ile Gly Val Ile Asp Ile Gly Ser Asp Gln Leu Asp Arg
   290 295 300
   Ser Leu Lys Gly Leu Val Pro Asp Glu Asp Val Asp Asn Leu Val Asn
   305 310 315 320
   Thr Ile His Ala Asn Thr His Gly Glu Ser Gln Ala Ala Thr Gly Ala
   325 330 335
   Ala Ala Ser Asn Leu Asp Leu Tyr Gln Val Asn Ser Thr Tyr Tyr Ser
   340 345 350
   Ala Leu Gly Cys Asn Asp Gln His Tyr Ile Ala Ala Arg Ala Val Gln
   355 360 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Ser Asn Gly Arg Asp
   385                 390                 395                 400
   Ile Asn Arg His Tyr Tyr Ser Thr Ala Glu Ile Asp Glu Asn Leu Lys
                   405                 410                 415
   Arg Pro Val Val Lys Ala Leu Asn Ala Leu Ala Lys Phe Arg Asn Glu
               420                 425                 430
   Leu Asp Ala Phe Asp Gly Thr Phe Ser Tyr Thr Thr Asp Asp Asp Thr
           435                 440                 445
   Ser Ile Ser Phe Thr Trp Arg Gly Glu Thr Ser Gln Ala Thr Leu Thr
       450                 455                 460
   Phe Glu Pro Lys Arg Gly Leu Gly Val Glu Asn Thr Thr Pro Val Ala
   465                 470                 475                 480
   Met Leu Glu Trp Glu Asp Ser Ala Gly Asp His Arg Ser Asp Asp Leu
                   485                 490                 495
   Ile Ala Asn Pro Pro Val Val Ala
               500
<210> 3
   <211> 504
   <212> PRT
   <213> artificial
<220>
   <223> modified sequence
<400> 3
   Met Lys Asn Lys Val Gln Leu Ile Thr Tyr Ala Asp Arg Leu Gly Asp
   1               5                   10                  15
   Gly Thr Ile Lys Ser Met Thr Asp Ile Leu Arg Thr Arg Phe Asp Gly
               20                  25                  30
   Val Tyr Asp Gly Val His Ile Leu Pro Phe Phe Thr Pro Phe Asp Gly
           35                  40                  45
   Ala Asp Ala Gly Phe Asp Pro Ile Asp His Thr Lys Val Asp Glu Arg
       50                  55                  60
   Leu Gly Ser Trp Asp Asp Val Ala Glu Leu Ser Lys Thr His Asn Ile
   65                  70                  75                  80
   Met Val Asp Ala Ile Val Asn His Met Ser Trp Glu Ser Lys Gln Phe
                   85                  90                  95
   Gln Asp Val Leu Ala Lys Gly Glu Glu Ser Glu Tyr Tyr Pro Met Phe
               100                 105                 110
   Leu Thr Met Ser Ser Val Phe Pro Asn Gly Ala Thr Glu Glu Asp Leu
           115                 120                 125
   Ala Gly Ile Tyr Arg Pro Arg Pro Gly Leu Pro Phe Thr His Tyr Lys
       130                 135                 140
   Phe Ala Gly Lys Thr Arg Tyr Val Trp Val Ser Phe Thr Pro Gln Gln
   145                 150                 155                 160
   Val Asp Ile Asp Thr Asp Ser Asp Lys Gly Trp Glu Tyr Leu Met Ser
                   165                 170                 175
   Ile Phe Asp Gln Met Ala Ala Ser His Val Ser Tyr Ile Arg Leu Asp
               180                 185                 190
   Ala Val Gly Tyr Gly Ala Lys Glu Ala Gly Thr Ser Cys Phe Met Thr
           195                 200                 205
   Pro Lys Thr Phe Lys Leu Ile Ser Arg Leu Arg Glu Glu Gly Val Lys
       210                 215                 220
   Arg Gly Leu Glu Ile Leu Ile Glu Val His Ser Tyr Tyr Lys Lys Gln
   225                 230                 235                 240
   Val Glu Ile Ala Ser Lys Val Asp Arg Val Tyr Asp Phe Ala Leu Pro
                   245                 250                 255
   Pro Leu Leu Leu His Ala Leu Ser Thr Gly His Val Glu Pro Val Ala
               260                 265                 270
   His Trp Thr Asp Ile Arg Pro Asn Asn Ala Val Thr Val Leu Asp Thr
           275                 280                 285
   His Asp Gly Ile Gly Val Ile Asp Ile Gly Ser Asp Gln Leu Asp Arg
       290                 295                 300
   Ser Leu Lys Gly Leu Val Pro Asp Glu Asp Val Asp Asn Leu Val Asn
   305                 310                 315                 320
   Thr Ile His Ala Asn Thr His Gly Glu Ser Gln Ala Ala Thr Gly Ala
                   325                 330                 335
   Ala Ala Ser Asn Leu Asp Leu Tyr Gln Val Asn Ser Thr Tyr Tyr Ser
               340                 345                 350
   Ala Leu Gly Cys Asn Asp Gln His Tyr Ile Ala Ala Arg Ala Val Gln
           355                 360                 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Ser Asn Gly Arg Asp
   385                 390                 395                 400
   Ile Asn Arg His Tyr Tyr Ser Thr Ala Glu Ile Asp Glu Asn Leu Lys
                   405                 410                 415
   Arg Pro Val Val Lys Ala Leu Asn Ala Leu Ala Lys Phe Arg Asn Glu
               420                 425                 430
   Leu Asp Ala Phe Asp Gly Thr Phe Ser Tyr Thr Thr Asp Asp Asp Thr
           435                 440                 445
   Ser Ile Ser Phe Thr Trp Arg Gly Glu Thr Ser Gln Ala Thr Leu Thr
       450                 455                 460
   Phe Glu Pro Lys Arg Gly Leu Gly Val Glu Asn Thr Thr Pro Val Ala
   465                 470                 475                 480
   Met Leu Glu Trp Glu Asp Ser Ala Gly Asp His Arg Ser Asp Asp Leu
                   485                 490                 495
   Ile Ala Asn Pro Pro Val Val Ala
               500
<210> 4
   <211> 504
   <212> PRT
   <213> artificial
<220>
   <223> modified sequence
<400> 4
   Met Lys Asn Lys Val Gln Leu Ile Thr Tyr Ala Asp Arg Leu Gly Asp
   1               5                   10                  15
   Gly Thr Ile Lys Gly Met Thr Asp Ile Leu Arg Thr Arg Phe Asp Gly
   20 25 30
   Val Tyr Asp Gly Val His Ile Leu Pro Phe Phe Thr Pro Phe Asp Gly
   35 40 45
   Ala Asp Ala Gly Phe Asp Pro Ile Asp His Thr Lys Val Asp Glu Arg
   50 55 60
   Leu Gly Ser Trp Asp Asp Val Ala Glu Leu Ser Lys Thr His Asn Ile
   65 70 75 80
   Met Val Asp Ala Ile Val Asn His Met Ser Trp Glu Ser Lys Gln Phe
   85 90 95
   Gln Asp Val Leu Ala Lys Gly Glu Glu Ser Glu Tyr Tyr Pro Met Phe
   100 105 110
   Leu Thr Met Ser Ser Val Phe Pro Asn Gly Ala Thr Glu Glu Asp Leu
   115 120 125
   Ala Gly Ile Tyr Arg Pro Arg Pro Gly Leu Pro Phe Thr His Tyr Lys
   130 135 140
   Phe Ala Gly Lys Thr Arg Leu Val Trp Val Ser Phe Thr Pro Gln Gln
   145 150 155 160
   Val Asp Ile Asp Thr Asp Ser Asp Lys Gly Trp Glu Tyr Leu Met Ser
   165 170 175
   Ile Phe Asp Gln Met Ala Ala Ser Gly Val Ser Tyr Ile Arg Leu Asp
   180 185 190
   Ala Val Gly Tyr Gly Ala Lys Glu Ala Gly Thr Ser Cys Phe Met Thr
   195 200 205
   Pro Lys Thr Phe Lys Leu Ile Ser Arg Leu Arg Glu Glu Gly Val Lys
   210 215 220
   Arg Gly Leu Glu Ile Leu Ile Glu Val His Ser Tyr Tyr Lys Lys Gln
   225 230 235 240
   Val Glu Ile Ala Ser Lys Val Asp Arg Val Tyr Asp Phe Ala Leu Pro
   245 250 255
   Pro Leu Leu Leu His Ala Leu Ser Thr Gly His Val Glu Pro Val Ala
   260 265 270
   His Trp Thr Asp Ile Arg Pro Asn Asn Ala Val Thr Val Leu Asp Thr
   275 280 285
   His Asp Gly Ile Gly Val Val Asp Ile Gly Ser Asp Gln Leu Asp Arg
   290 295 300
   Ser Leu Lys Gly Leu Val Pro Asp Glu Asp Val Asp Asn Leu Val Asn
   305 310 315 320
   Thr Ile His Ala Asn Thr His Gly Glu Ser Gln Ala Ala Thr Gly Ala
   325 330 335
   Ala Ala Ser Asn Leu Asp Leu Tyr Gln Val Asn Ser Thr Tyr Tyr Ser
   340 345 350
   Ala Leu Gly Cys Asn Asp Gln His Tyr Ile Ala Ala Arg Ala Val Gln
   355 360 365
   Phe Phe Leu Pro Gly Val Pro Gln Val Tyr Tyr Val Gly Ala Leu Ala
   370 375 380
   Gly Lys Asn Asp Met Glu Leu Leu Arg Lys Thr Asn Asn Gly Arg Asp
   385 390 395 400
   Ile Asn Arg His Tyr Tyr Ser Thr Ala Glu Ile Asp Glu Asn Leu Lys
   405 410 415
   Arg Pro Val Val Lys Ala Leu Asn Ala Leu Ala Lys Phe Arg Asn Glu
   420 425 430
   Leu Asp Ala Phe Asp Gly Thr Phe Ser Tyr Thr Thr Asp Asp Asp Thr
   435 440 445
   Ser Ile Ser Phe Thr Trp Arg Gly Glu Thr Ser Gln Ala Thr Leu Thr
   450 455 460
   Phe Glu Pro Lys Arg Gly Leu Gly Val Glu Asn Thr Thr Pro Val Ala
   465 470 475 480
   Met Leu Glu Trp Glu Asp Ser Ala Gly Asp His Arg Ser Asp Asp Leu
   485 490 495
   Ile Ala Asn Pro Pro Val Val Ala
   500
<210> 5
   <211> 504
   <212> PRT
   <213> artificial
<220>
   <223> modified sequence
<400> 5
   Met Lys Asn Lys Val Gln Leu Ile Thr Tyr Ala Asp Arg Leu Gly Asp
   1               5                   10                  15
   Gly Thr Ile Lys Ser Met Thr Asp Ile Leu Arg Thr Arg Phe Asp Gly
               20                  25                  30
   Val Tyr Asp Gly Val His Ile Leu Pro Phe Phe Thr Pro Phe Asp Gly
           35                  40                  45
   Ala Asp Ala Gly Phe Asp Pro Ile Asp His Thr Lys Val Asp Glu Arg
       50                  55                  60
   Leu Gly Ser Trp Asp Asp Val Ala Glu Leu Ser Lys Thr His Asn Ile
   65                  70                  75                  80
   Met Val Asp Ala Ile Val Asn His Met Ser Trp Glu Ser Lys Gln Phe
                   85                  90                  95
   Gln Asp Val Leu Ala Lys Gly Glu Glu Ser Glu Tyr Tyr Pro Met Phe
               100                 105                 110
   Leu Thr Met Ser Ser Val Phe Pro Asn Gly Ala Thr Glu Glu Asp Leu
           115                 120                 125
   Ala Gly Ile Tyr Arg Pro Arg Pro Gly Leu Pro Phe Thr His Tyr Lys
       130                 135                 140
   Phe Ala Gly Lys Thr Arg Leu Val Trp Val Ser Phe Thr Pro Gln Gln
   145                 150                 155                 160
   Val Asp Ile Asp Thr Asp Ser Asp Lys Gly Trp Glu Tyr Leu Met Ser
                   165                 170                 175
   Ile Phe Asp Gln Met Ala Ala Ser Gly Val Ser Tyr Ile Arg Leu Asp
               180                 185                 190
   Ala Val Gly Tyr Gly Ala Lys Glu Ala Gly Thr Ser Cys Phe Met Thr
           195                 200                 205
   Pro Lys Thr Phe Lys Leu Ile Ser Arg Leu Arg Glu Glu Gly Val Lys
       210                 215                 220
   Arg Gly Leu Glu Ile Leu Ile Glu Val His Ser Tyr Tyr Lys Lys Gln
   225                 230                 235                 240
   Val Glu Ile Ala Ser Lys Val Asp Arg Val Tyr Asp Phe Ala Leu Pro
                   245                 250                 255
   Pro Leu Leu Leu His Ala Leu Ser Thr Gly His Val Glu Pro Val Ala
               260                 265                 270
   His Trp Thr Asp Ile Arg Pro Asn Asn Ala Val Thr Val Leu Asp Thr
           275                 280                 285
   His Asp Gly Ile Gly Val Val Asp Ile Gly Ser Asp Gln Leu Asp Arg
       290                 295                 300
   Ser Leu Lys Gly Leu Val Pro Asp Glu Asp Val Asp Asn Leu Val Asn
   305                 310                 315                 320
   Thr Ile His Ala Asn Thr His Gly Glu Ser Gln Ala Ala Thr Gly Ala
                   325                 330                 335
   Ala Ala Ser Asn Leu Asp Leu Tyr Gln Val Asn Ser Thr Tyr Tyr Ser
               340                 345                 350
   Ala Leu Gly Cys Asn Asp Gln His Tyr Ile Ala Ala Arg Ala Val Gln
           355                 360                 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Asn Asn Gly Arg Asp
   385                 390                 395                 400
   Ile Asn Arg His Tyr Tyr Ser Thr Ala Glu Ile Asp Glu Asn Leu Lys
                   405                 410                 415
   Arg Pro Val Val Lys Ala Leu Asn Ala Leu Ala Lys Phe Arg Asn Glu
               420                 425                 430
   Leu Asp Ala Phe Asp Gly Thr Phe Ser Tyr Thr Thr Asp Asp Asp Thr
           435                 440                 445
   Ser Ile Thr Phe Thr Trp Arg Gly Glu Thr Ser Gln Ala Thr Leu Thr
       450                 455                 460
   Phe Glu Pro Lys Arg Gly Leu Gly Val Glu Asn Thr Thr Pro Val Ala
   465                 470                 475                 480
   Met Leu Glu Trp Glu Asp Ser Ala Gly Asp His Arg Ser Asp Asp Leu
   485 490 495
   Ile Ala Asn Pro Pro Val Val Ala
   500
<210> 6
   <211> 504
   <212> PRT
   <213> artificial
<220>
   <223> modified sequence
<400> 6
   Met Lys Asn Lys Val Gln Leu Ile Thr Tyr Ala Asp Arg Leu Gly Asp
   1 5 10 15
   Gly Thr Ile Lys Ser Met Thr Asp Ile Leu Arg Thr Arg Phe Asp Gly
   20 25 30
   Val Tyr Asp Gly Val His Ile Leu Pro Phe Phe Thr Pro Phe Asp Gly
   35 40 45
   Ala Asp Ala Gly Phe Asp Pro Ile Asp His Thr Lys Val Asp Glu Arg
   50 55 60
   Leu Gly Ser Trp Asp Asp Val Ala Glu Leu Ser Lys Thr His Asn Ile
   65 70 75 80
   Met Val Asp Ala Ile Val Asn His Met Ser Trp Glu Ser Lys Gln Phe
   85 90 95
   Gln Asp Val Leu Ala Lys Gly Glu Glu Ser Glu Tyr Tyr Pro Met Phe
   100 105 110
   Leu Thr Met Ser Ser Val Phe Pro Asn Gly Ala Thr Glu Glu Asp Leu
   115 120 125
   Ala Gly Ile Tyr Arg Pro Arg Pro Gly Leu Pro Phe Thr His Tyr Lys
   130 135 140
   Phe Ala Gly Lys Thr Arg Leu Val Trp Val Ser Phe Thr Pro Gln Gln
   145 150 155 160
   Val Asp Ile Asp Thr Asp Ser Asp Lys Gly Trp Glu Tyr Leu Met Ser
   165 170 175
   Ile Phe Asp Gln Met Ala Ala Ser Gly Val Ser Tyr Ile Arg Leu Asp
               180                 185                 190
   Ala Val Gly Tyr Gly Ala Lys Glu Ala Gly Thr Ser Cys Phe Met Thr
           195                 200                 205
   Pro Lys Thr Phe Lys Leu Ile Ser Arg Leu Arg Glu Glu Gly Val Lys
       210                 215                 220
   Arg Gly Leu Glu Ile Leu Ile Glu Val His Ser Tyr Tyr Lys Lys Gln
   225                 230                 235                 240
   Val Glu Ile Ala Ser Lys Val Asp Arg Val Tyr Asp Phe Ala Leu Pro
                   245                 250                 255
   Pro Leu Leu Leu His Ala Leu Ser Thr Gly His Val Glu Pro Val Ala
               260                 265                 270
   His Trp Thr Asp Ile Arg Pro Asn Asn Ala Val Thr Val Leu Asp Thr
           275                 280                 285
   His Asp Gly Ile Gly Val Val Asp Ile Gly Ser Asp Gln Leu Asp Arg
       290                 295                 300
   Ser Leu Lys Gly Leu Val Pro Asp Glu Asp Val Asp Asn Leu Val Asn
   305                 310                 315                 320
   Thr Ile His Ala Asn Thr His Gly Glu Ser Gln Ala Ala Thr Gly Ala
                   325                 330                 335
   Ala Ala Ser Asn Leu Asp Leu Tyr Gln Val Asn Ser Thr Tyr Tyr Ser
               340                 345                 350
   Ala Leu Gly Cys Asn Asp Gln His Tyr Ile Ala Ala Arg Ala Val Gln
           355                 360                 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Asn Asn Gly Arg Asp
   385                 390                 395                 400
   Ile Asn Arg His Tyr Tyr Ser Thr Ala Glu Ile Asp Glu Asn Leu Lys
                   405                 410                 415
   Arg Pro Val Val Lys Ala Leu Asn Ala Leu Ala Lys Phe Arg Asn Glu
               420                 425                 430
   Leu Asp Ala Phe Asp Gly Thr Phe Ser Tyr Thr Thr Asp Asp Asp Thr
           435                 440                 445
   Ser Ile Ser Phe Thr Trp Arg Gly Glu Thr Ser Gln Ala Thr Leu Thr
       450                 455                 460
   Phe Glu Pro Lys Arg Gly Leu Gly Val Asp Asn Ala Thr Pro Val Ala
   465                 470                 475                 480
   Met Leu Glu Trp Glu Asp Ser Ala Gly Asp His Arg Ser Asp Asp Leu
                   485                 490                 495
   Ile Ala Asn Pro Pro Val Val Ala
               500
<210> 7
   <211> 504
   <212> PRT
   <213> artificial
<220>
   <223> modified sequence
<400> 7
   Met Lys Asn Lys Val Gln Leu Ile Thr Tyr Ala Asp Arg Leu Gly Asp
   1               5                   10                  15
   Gly Thr Ile Lys Ser Met Thr Asp Ile Leu Arg Thr Arg Phe Asp Gly
               20                  25                  30
   Val Tyr Asp Gly Val His Ile Leu Pro Phe Phe Thr Pro Phe Asp Gly
           35                  40                  45
   Ala Asp Ala Gly Phe Asp Pro Ile Asp His Thr Lys Val Asp Glu Arg
       50                  55                  60
   Leu Gly Ser Trp Asp Asp Val Ala Glu Leu Ser Lys Thr His Asn Ile
   65                  70                  75                  80
   Met Val Asp Ala Ile Val Asn His Met Ser Trp Glu Ser Lys Gln Phe
                   85                  90                  95
   Gln Asp Val Leu Ala Lys Gly Glu Glu Ser Glu Tyr Tyr Pro Met Phe
               100                 105                 110
   Leu Thr Met Ser Ser Val Phe Pro Asn Gly Ala Thr Glu Glu Asp Leu
           115                 120                 125
   Ala Gly Ile Tyr Arg Pro Arg Pro Gly Leu Pro Phe Thr His Tyr Lys
       130                 135                 140
   Phe Ala Gly Lys Thr Arg Leu Val Trp Val Ser Phe Thr Pro Gln Gln
   145                 150                 155                 160
   Val Asp Ile Asp Thr Asp Ser Asp Lys Gly Trp Glu Tyr Leu Met Ser
                   165                 170                 175
   Ile Phe Asp Gln Met Ala Ala Ser His Val Ser Tyr Ile Arg Leu Asp
               180                 185                 190
   Ala Val Gly Tyr Gly Ala Lys Glu Ala Gly Thr Ser Cys Phe Met Thr
           195                 200                 205
   Pro Lys Thr Phe Lys Leu Ile Ser Arg Leu Arg Glu Glu Gly Val Lys
       210                 215                 220
   Arg Gly Leu Glu Ile Leu Ile Glu Val His Ser Tyr Tyr Lys Lys Gln
   225                 230                 235                 240
   Val Glu Ile Ala Ser Lys Val Asp Arg Val Tyr Asp Phe Ala Leu Pro
                   245                 250                 255
   Pro Leu Leu Leu His Ala Leu Ser Thr Gly His Val Glu Pro Val Ala
               260                 265                 270
   His Trp Thr Asp Ile Arg Pro Asn Asn Ala Val Thr Val Leu Asp Thr
           275                 280                 285
   His Asp Gly Ile Gly Val Ile Asp Ile Gly Ser Asp Gln Leu Asp Arg
       290                 295                 300
   Ser Leu Lys Gly Leu Val Pro Asp Glu Asp Val Asp Asn Leu Val Asn
   305                 310                 315                 320
   Thr Ile His Ala Asn Thr His Gly Glu Ser Gln Ala Ala Thr Gly Ala
                   325                 330                 335
   Ala Ala Ser Asn Leu Asp Leu Tyr Gln Val Asn Ser Thr Tyr Tyr Ser
               340                 345                 350
   Ala Leu Gly Cys Asn Asp Gln His Tyr Ile Ala Ala Arg Ala Val Gln
           355                 360                 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Ser Asn Gly Arg Asp
   385 390 395 400
   Ile Asn Arg His Tyr Tyr Ser Thr Ala Glu Ile Asp Glu Asn Leu Lys
   405 410 415
   Arg Pro Val Val Lys Ala Leu Asn Ala Leu Ala Lys Phe Arg Asn Glu
   420 425 430
   Leu Asp Ala Phe Asp Gly Thr Phe Ser Tyr Thr Thr Asp Asp Asp Thr
   435 440 445
   Ser Ile Ser Phe Thr Trp Arg Gly Glu Thr Ser Gln Ala Thr Leu Thr
   450 455 460
   Phe Glu Pro Lys Arg Gly Leu Gly Val Glu Asn Thr Thr Pro Val Ala
   465 470 475 480
   Met Leu Glu Trp Glu Asp Ser Ala Gly Asp His Arg Ser Asp Asp Leu
   485 490 495
   Ile Ala Asn Pro Pro Val Val Ala
   500
<210> 8
   <211> 504
   <212> PRT
   <213> artificial
<220>
   <223> modified sequence
<400> 8
   Met Lys Asn Lys Val Gln Leu Ile Thr Tyr Ala Asp Arg Leu Gly Asp
   1 5 10 15
   Gly Thr Ile Lys Ser Met Thr Asp Ile Leu Arg Thr Arg Phe Asp Gly
   20 25 30
   Val Tyr Asp Gly Val His Ile Leu Pro Phe Phe Thr Pro Phe Asp Gly
   35 40 45
   Ala Asp Ala Gly Phe Asp Pro Ile Asp His Thr Lys Val Asp Glu Arg
   50 55 60
   Leu Gly Ser Trp Asp Asp Val Ala Glu Leu Ser Lys Thr His Asn Ile
   65 70 75 80
   Met Val Asp Ala Ile Val Asn His Met Ser Trp Glu Ser Lys Gln Phe
                   85                  90                  95
   Gln Asp Val Leu Ala Lys Gly Glu Glu Ser Glu Tyr Tyr Pro Met Phe
               100                 105                 110
   Leu Thr Met Ser Ser Val Phe Pro Asn Gly Ala Thr Glu Glu Asp Leu
           115                 120                 125
   Ala Gly Ile Tyr Arg Pro Arg Pro Gly Leu Pro Phe Thr His Tyr Lys
       130                 135                 140
   Phe Ala Gly Lys Thr Arg Leu Val Trp Val Ser Phe Thr Pro Gln Gln
   145                 150                 155                 160
   Val Asp Ile Asp Thr Asp Ser Asp Lys Gly Trp Glu Tyr Leu Met Ser
                   165                 170                 175
   Ile Phe Asp Gln Met Ala Ala Ser Gly Val Ser Tyr Ile Arg Leu Asp
               180                 185                 190
   Ala Val Gly Tyr Gly Ala Lys Glu Ala Gly Thr Ser Cys Phe Met Thr
           195                 200                 205
   Pro Lys Thr Phe Lys Leu Ile Ser Arg Leu Arg Glu Glu Gly Val Lys
       210                 215                 220
   Arg Gly Leu Glu Ile Leu Ile Glu Val His Ser Tyr Tyr Lys Lys Gln
   225                 230                 235                 240
   Val Glu Ile Ala Ser Lys Val Asp Arg Val Tyr Asp Phe Ala Leu Pro
                   245                 250                 255
   Pro Leu Leu Leu His Ala Leu Ser Thr Gly His Val Glu Pro Val Ala
               260                 265                 270
   His Trp Thr Asp Ile Arg Pro Asn Asn Ala Val Thr Val Leu Asp Thr
           275                 280                 285
   His Asp Gly Ile Gly Val Val Asp Ile Gly Ser Asp Gln Leu Asp Arg
       290                 295                 300
   Ser Leu Lys Gly Leu Val Pro Asp Glu Asp Val Asp Asn Leu Val Asn
   305                 310                 315                 320
   Thr Ile His Ala Asn Thr His Gly Glu Ser Gln Ala Ala Thr Gly Ala
                   325                 330                 335
   Ala Ala Ser Asn Leu Asp Leu Tyr Gln Val Asn Ser Thr Tyr Tyr Ser
               340                 345                 350
   Ala Leu Gly Cys Asn Asp Gln His Tyr Ile Ala Ala Arg Ala Val Gln
           355                 360                 365
   Phe Phe Leu Pro Gly Val Pro Gln Val Tyr Tyr Val Gly Ala Leu Ala
       370                 375                 380
   Gly Lys Asn Asp Met Glu Leu Leu Arg Lys Thr Asn Asn Gly Arg Asp
   385                 390                 395                 400
   Ile Asn Arg His Tyr Tyr Ser Thr Ala Glu Ile Asp Glu Asn Leu Lys
                   405                 410                 415
   Arg Pro Val Val Lys Ala Leu Asn Ala Leu Ala Lys Phe Arg Asn Glu
               420                 425                 430
   Leu Asp Ala Phe Asp Gly Thr Phe Ser Tyr Thr Thr Asp Asp Asp Thr
           435                 440                 445
   Ser Ile Ser Phe Thr Trp Arg Gly Glu Thr Ser Gln Ala Thr Leu Thr
       450                 455                 460
   Phe Glu Pro Lys Arg Gly Leu Gly Val Glu Asn Thr Thr Pro Val Ala
   465                 470                 475                 480
   Met Leu Glu Trp Glu Asp Ser Ala Gly Asp His Arg Ser Asp Asp Leu
                   485                 490                 495
   Ile Ala Asn Pro Pro Val Val Ala
               500